# EUROPÄISCHE PATENTSCHRIFT

(11) **EP 3 102 254 B1**
(45) Veröffentlichungstag und Bekanntmachung des Hinweises auf die Patenterteilung: **02.10.2019**
(21) Anmeldenummer: 15701676.7
(22) Anmeldetag: 31.01.2015
(51) Int. Cl.: A61M 5/20, A61M 5/315, A61M 5/24

(54) **INJEKTIONSGERÄT**
INJECTION DEVICE
DISPOSITIF D'INJECTION

(30) Priorität: 05.02.2014 DE 202014001136 U
(43) Veröffentlichungstag der Anmeldung: 14.12.2016
(73) Patentinhaber: Haselmeier AG, 9001 St. Gallen (CH)
(72) Erfinder: KEITEL, Joachim, 73728 Esslingen (DE)
(74) Vertreter: Reinhardt, Annette
(86) Internationale Anmeldenummer: PCT/EP2015/000184
(87) Internationale Veröffentlichungsnummer: WO 2015/117741

(56) Entgegenhaltungen:
- EP-A2- 0 268 191
- WO-A1-2013/117332
- DE-A1-102005 060 928

## Beschreibung

Die Erfindung betrifft ein Injektionsgerät der im Oberbegriff des Anspruchs 1 angegebenen Gattung.

Aus der WO 2013/117332 A1 ist ein Injektionsgerät bekannt, dessen Dosierorgan im Gehäuse drehbar und axial fest gehalten ist. Das Dosierorgan dreht sich beim Einstellen einer auszupressenden Menge an Injektionsflüssigkeit in einer ersten Drehrichtung und beim Auspressen der Injektionsflüssigkeit in Gegenrichtung. Zwischen dem Dosierorgan und dem Gehäuse wirkt eine Rasteinrichtung.

Aus der DE 10 2005 060 928 A1 ist ein Injektionsgerät bekannt, das ein Dosierglied besitzt, das über eine Gewindeverbindung im Gehäuse gehalten ist. Beim Einstellen einer auszupressenden Menge an Injektionsflüssigkeit bewegt sich das Dosierglied in distale Richtung und dreht sich aufgrund der Gewindeverbindung gegenüber dem Gehäuse. Beim Auspressen von Injektionsflüssigkeit bewegt sich das Dosierglied in proximale Richtung und dreht sich in Gegenrichtung.

Aus der EP 1 610 848 B2 ist ein Injektionsgerät bekannt, das als Einstellteil ein Skalenrohr besitzt. Beim Einstellen einer auszupressenden Menge einer Injektionsflüssigkeit bewegt sich das Skalenrohr in distale Richtung. Beim Auspressen der auszupressenden Menge einer Injektionsflüssigkeit bewegt sich das Skalenrohr in Gegenrichtung. Das Skalenrohr ist über eine Gewindeverbindung mit dem Gehäuse verbunden, so dass sich das Skalenrohr zusätzlich zur Bewegung in distale oder proximale Richtung auch gegenüber dem Gehäuse dreht. Das Injektionsgerät besitzt außerdem eine Rasteinrichtung, die zwischen einem Gewindeteil und dem Gehäuse wirkt. Beim Einstellen der auszupressenden Menge an Injektionsflüssigkeit dreht sich das Gewindeteil gegenüber dem Gehäuse. Beim Auspressen der auszupressenden Menge an Injektionsflüssigkeit ist das Gewindeteil im Gehäuse in axialer Richtung geführt, so dass die Rasteinrichtung beim Auspressen einer Dosis nicht wirksam ist und keine Klicks der Rasteinrichtung zu hören sind.

Das aus der EP 1 680 848 B2 bekannte Injektionsgerät besitzt feste Dosierschritte. Werden für eine Therapie beispielsweise einzustellende Mengen an Injektionsflüssigkeit von 0,20 ml und 0,25 ml benötigt, so sind bekannte Injektionsgeräte so ausgelegt, dass Dosierschritte von höchstens 0,05 ml einstellbar sind. Dies bedeutet zum einen, dass der Anwender mehrere Rastschritte überwinden muss, bis er die kleinste, für die Therapie vorgesehene Dosis erreicht. Zum anderen ist bei einem kleinsten festen Dosierschritt von beispielsweise 0,05 ml die beim Priming-Vorgang zu verwerfende Menge an Injektionsflüssigkeit vergleichsweise groß. Für den Priming-Vorgang wären deshalb deutlich kleinere Dosierschritte wünschenswert. Dies führt allerdings zu einer deutlich erhöhten Zahl von Raststellungen, die der Bediener beim Einstellen der Dosis überwinden muss.

Der vorliegenden Erfindung liegt die Aufgabe zugrunde, ein Injektionsgerät der gattungsgemäßen Art zu schaffen, das die Anordnung mehrerer Raststellungen in unterschiedlichen Abständen ermöglicht.

Diese Aufgabe wird durch ein Injektionsgerät mit den Merkmalen des Anspruchs 1 gelöst.

Die vorliegende Erfindung sieht vor, dass jeder Raststellung eine eindeutige Drehlage des Einstellteils gegenüber dem Gehäuse zugeordnet ist. Dadurch können die benötigten Raststellungen mit unterschiedlichen Abständen zueinander angeordnet werden. Beispielsweise könnte für die eingangs beispielhaft beschriebene Therapie ein Injektionsgerät vorgesehen werden, das genau drei Raststellungen bei 0,01 ml für den Priming-Vorgang und 0,20 ml und 0,25 ml für die zu injizierenden Dosen bereitstellt. Dadurch wird die Bedienung des Injektionsgeräts erheblich vereinfacht. Dadurch, dass das Zustellteil, der Schieber und das Einstellteil über unterschiedliche Gewindeverbindungen in axialer Richtung bewegt werden, sind für das Einstellteil und Schieber und Zustellteil unterschiedliche axiale Wege möglich. Das Injektionsgerät kann so ausgelegt werden, dass die Injektion vom Bediener manuell durchgeführt werden kann, so dass der Bediener die Injektionsgeschwindigkeit selbst steuern kann.

Die Rasteinrichtung wirkt zwischen dem Einstellteil und dem Gehäuse. Dabei muss die Rasteinrichtung nicht von dem Einstellteil und dem Gehäuse gebildet sein, sondern kann auch an Bauteilen angeordnet sein, die drehfest mit dem Einstellteil bzw. dem Gehäuse verbunden sind. Die Rasteinrichtung ist demnach von dem Einstellteil oder einem drehfest mit dem Einstellteil verbundenen Bauteil und von dem Gehäuse oder einem drehfest mit dem Gehäuse verbundenen Bauteil gebildet.

Bei dem Injektionsgerät gemäß der EP 1 610 848 B2 verändert sich die relative radiale Lage des Gewindeteils gegenüber dem Skalenrohr bei jeder Injektion. Beim Einstellen einer Dosis drehen sich Skalenrohr und Gewindeteil gegenüber dem Gehäuse. Beim Auspressen der auszupressenden Menge an Injektionsflüssigkeit dreht sich das Skalenrohr zurück, während das Gewindeteil drehfest und axial beweglich im Gehäuse geführt ist. Dadurch ist die Drehlage des Gewindeteils im Gehäuse bei einer vorgegebenen einzustellenden Dosis unbestimmt und kann sich bei jedem Injektionsvorgang ändern. Die vorliegende Erfindung sieht dagegen vor, dass jeder Raststellung eine eindeutige Drehlage des Einstellteils gegenüber dem Gehäuse zugeordnet ist. Dadurch können die Raststellungen in unterschiedlichen Abständen zueinander angeordnet werden. Beispielsweise können Raststellungen, die nicht vorgesehenen Mengen an Injektionsflüssigkeit zugeordnet sind, entfallen.

Vorteilhaft umfasst die Rasteinrichtung ein Rastteil, das unabhängig vom Einstellteil in Richtung der Längsmittelachse des Injektionsgeräts verschiebbar ist und das drehfest mit dem Gehäuse verbunden ist. Vorteilhaft ist an dem Rastteil mindestens ein erstes Rastelement angeordnet. Durch die Anordnung des Rastelements an einem axial verschiebbaren Rastteil kann das Rastelement durch Verschieben in Richtung der Längsmittelachse des Injektionsgeräts in eine Stellung gebracht werden, in der es nicht wirksam ist. An dem Einstellteil ist vorteilhaft mindestens ein zweites Rastelement angeordnet. Das mindestens eine erste Rastelement und das mindestens eine zweite Rastelement definieren vorteilhaft in einer ersten axialen Stellung von Rastteil und Einstellteil die mindestens eine Raststellung und sind in mindestens einer zweiten axialen Stellung von Rastteil und Einstellteil unabhängig von der relativen Drehlage des Einstellteils zum Rastteil außer Eingriff. Dadurch kann sich das Einstellteil gegenüber dem Rastteil beim Auspressen einer auszupressenden Menge an Injektionsflüssigkeit zurückstellen, ohne dass die Raststellungen hörbar und spürbar für den Benutzer sind und vom Benutzer zu überwinden sind. Dadurch ergibt sich eine einfache und ergonomische Bedienung. Vorteilhaft besitzt das Injektionsgerät eine Feder, die das Rastteil in Richtung auf die erste axiale Stellung vorspannt.

Vorteilhaft ist das Einstellteil drehfest mit dem Schieber verbunden. Der Schieber wirkt beim Auspressen von Injektionsflüssigkeit aus dem Behälter vorteilhaft derart auf das Zustellteil, dass der Schieber bei einer Bewegung in proximale Richtung das Zustellteil in proximaler Richtung verschiebt. Der Dosierkolben ist vorteilhaft drehfest im Gehäuse gehalten. Beim Einstellen einer auszupressenden Menge an Injektionsflüssigkeit drehen sich Einstellteil, Schieber und Zustellteil vorteilhaft gegenüber dem Gehäuse über ihre jeweilige Gewindeverbindung. Beim Auspressen einer auszupressenden Menge an Injektionsflüssigkeit ist das Zustellteil vorteilhaft drehfest geführt und bewegt aufgrund seiner Bewegung in proximale Richtung den Dosierkolben mit. Das Einstellteil und der Schieber drehen sich beim Auspressen der auszupressenden Menge an Injektionsflüssigkeit zurück in ihre Ausgangslage.

Vorteilhaft besitzt das Injektionsgerät einen Mitnehmer, der drehfest mit dem Zustellteil verbunden ist. In Richtung der Längsmittelachse des Injektionsgeräts sind Mitnehmer und Zustellteil vorteilhaft relativ zueinander beweglich. Insbesondere besitzt das Injektionsgerät eine Kupplung, die in einer ersten Stellung das Einstellteil drehfest mit einem Mitnehmer verbindet, und in einer zweiten Stellung eine Relativdrehung des Einstellteils gegenüber dem Mitnehmer zulässt. Der Mitnehmer ist vorteilhaft drehfest mit dem Zustellteil verbunden. Das Verstellen der Kupplung von der ersten Stellung in die zweite Stellung erfolgt vorteilhaft durch Verschieben eines Betätigungsknopfes des Injektionsgerätes in proximale Richtung. Vorteilhaft ist das Rastteil in Richtung der Längsmittelachse derart an den Betätigungsknopf gekoppelt, dass eine Bewegung des Betätigungsknopfes in proximale Richtung eine Bewegung des Rastteils in proximale Richtung bewirkt.

Zum Einstellen der auszupressenden Menge an Injektionsflüssigkeit besitzt das Injektionsgerät vorteilhaft eine Stellhülse. Bei einer ersten Ausführungsform des Injektionsgeräts ist die Stellhülse fest mit dem Einstellteil verbunden. Die Stellhülse ist insbesondere einteilig mit dem Einstellteil verbunden. Vorteilhaft wirkt der Betätigungsknopf über ein Druckstück auf das Rastteil, wobei das Druckstück drehbar gegenüber dem Betätigungsknopf ist und drehfest mit dem Einstellteil verbunden ist. Das Einstellteil ist mit der Stellhülse vorteilhaft über einen Ringsteg verbunden, der mindestens eine Öffnung besitzt, durch die ein Drucksteg des Druckstücks ragt. Beim Auspressen der auszupressenden Menge an Injektionsflüssigkeit ist der Betätigungsknopf vorteilhaft drehfest geführt. Das Einstellteil dreht sich zusammen mit der Stellhülse zurück in seine Ausgangslage. Das Druckstück kann mit dem Einstellteil und der Stellhülse gegenüber dem Betätigungsknopf rotieren. Dadurch ergibt sich ein einfacher Aufbau. Vorteilhaft ist das Zustellteil über eine zweite Rasteinrichtung mit dem Gehäuse verbunden, wobei die Rasteinrichtung mindestens einen Längssteg umfasst, an dem das Zustellteil beim Auspressen einer eingestellten Menge an Injektionsflüssigkeit geführt ist. Die zweite Rasteinrichtung stellt sicher, dass sich das Zustellteil und der drehfest mit dem Zustellteil verbundene Betätigungsknopf beim Auspressen der eingestellten Menge an Injektionsflüssigkeit nicht gegenüber dem Gehäuse drehen und dadurch die auszupressende Menge an Injektionsflüssigkeit verringern können. Die zweite Rasteinrichtung ist gegenüber der ersten Rasteinrichtung zwischen Einstellteil und Rastteil vorteilhaft sehr schwach ausgelegt. Die Rastschritte der zweiten Rasteinrichtung sind so ausgelegt, dass alle einzustellenden Dosiswerte dem Vielfachen der Rastschritte entsprechen. Die Dosis für den Priming-Vorgang entspricht vorteilhaft mindestens dem Rastschritt der zweiten Rasteinrichtung.

Bei einer weiteren Ausführungsvariante kann vorgesehen sein, dass die Stellhülse fest mit dem Betätigungsknopf verbunden ist. Vorteilhaft besitzt das Injektionsgerät eine zweite Kupplung, die in der distalen Position des Betätigungsknopfes eine Relativdrehung des Mitnehmers gegenüber dem Rastteil zulässt und in einer proximalen Position des Betätigungsknopfes den Mitnehmer drehfest mit dem Rastteil verbindet. Dadurch ergibt sich ein einfacher Aufbau des Injektionsgerätes. Ein einfacher Aufbau der zweiten Kupplung ergibt sich, wenn der Mitnehmer und das Rastteil jeweils eine Verzahnung besitzen. Am Einstellteil ist vorteilhaft ein Kupplungsteil angeordnet, das eine Gegenverzahnung trägt. In der distalen Position des Betätigungsknopfes wirkt die Gegenverzahnung nur mit einer der Verzahnungen zusammen. In der proximalen Position des Betätigungsknopfes ist die Gegenverzahnung mit beiden Verzahnungen drehfest verbunden und verbindet dadurch Mitnehmer und Rastteil drehfest miteinander. Vorteilhaft ist das Kupplungsteil gegenüber dem Einstellteil drehbar und in Richtung der Längsmittelachse ortsfest am Einstellteil gehalten. Das Kupplungsteil kann dabei gegenüber dem Einstellteil geringfügig axial verschiebbar sein. Das Kupplungsteil muss derart ortsfest am Einstellteil gehalten sein, dass sichergestellt ist, dass das Kupplungsteil in der proximalen Position des Betätigungsknopfes sowohl mit der Verzahnung am Mitnehmer als auch mit der Verzahnung am Rastteil drehfest verbunden ist und in der distalen Position des Betätigungsknopfs nur mit einer der Verzahnungen. Ein einfacher Aufbau ergibt sich, wenn das Kupplungsteil in der distalen Position des Bedienknopfes nur mit der Verzahnung des Rastteils zusammenwirkt.

Vorteilhaft besitzt das Injektionsgerät eine Feder, die zwischen dem Schieber und dem Gehäuse wirkt und die den Schieber in der zweiten Drehrichtung vorspannt. Die Feder ist insbesondere eine Torsionsfeder. Die Vorspannung des Schiebers in der zweiten Drehrichtung bewirkt, dass sich der Schieber aufgrund der Federkraft zurück zur nächst niedrigen Raststellung der ersten Rasteinrichtung stellt, wenn keine vorgesehene Menge an Injektionsflüssigkeit eingestellt ist. Dadurch wird das Einstellen einer nicht vorgesehenen Menge an Injektionsflüssigkeit, der keine Raststellung zugeordnet ist, auf einfache Weise verhindert. Gleichzeitig unterstützt die Feder das Auspressen der eingestellten Menge an Injektionsflüssigkeit.

Ausführungsbeispiele der Erfindung werden im Folgenden anhand der Zeichnung erläutert. Es zeigen:
- Fig. 1: eine Seitenansicht eines Injektionsgerätes,
- Fig. 2: einen Schnitt durch das Injektionsgerät aus Fig. 1 entlang der Linie II-II in Fig. 1,
- Fig. 3: das Injektionsgerät aus Fig. 1 nach dem Einstellen der Maximaldosis,
- Fig. 4: einen Schnitt entlang der Linie IV-IV in Fig. 3,
- Fig. 5: das Injektionsgerät aus Fig. 1 bei eingestellter Maximaldosis nach dem Verschieben des Betätigungsknopfes in proximale Richtung,
- Fig. 6: einen Schnitt entlang der Linie VI-VI in Fig. 5,
- Fig. 7: eine Seitenansicht von Rastteil und Einstellteil in der in Fig. 1 gezeigten Stellung des Injektionsgerätes,
- Fig. 8: einen Schnitt entlang der Linie VIII-VIII in Fig. 7,
- Fig. 9: eine Seitenansicht von Einstellteil und Rastteil nach dem Betätigen des Betätigungsknopfes,
- Fig. 10: einen Schnitt entlang der Linie X-X in Fig. 9,
- Fig. 11: eine Seitenansicht des Einstellteils,
- Fig. 12: einen Schnitt entlang der Linie XII-XII in Fig. 11,
- Fig. 13: eine Seitenansicht auf das Einstellteil in Richtung des Pfeils XIII in Fig. 11,
- Fig. 14: einen Schnitt durch das Einstellteil entlang der Linie XIV-XIV in Fig. 11,
- Fig. 15: einen Schnitt durch das Einstellteil entlang der Linie XV-XV in Fig. 14,
- Fig. 16: eine Draufsicht auf das Einstellteil in Richtung des Pfeils XVI in Fig. 13,
- Fig. 17 und 18: perspektivische Darstellungen des Rastteils,
- Fig. 19: eine Seitenansicht des Rastteils aus den Figuren 17 und 18,
- Fig. 20: eine Seitenansicht des Rastteils in Richtung des Pfeils XX in Fig. 19,
- Fig. 21: einen Schnitt durch das Rastteil entlang der Linie XXI-XXI in Fig. 20,
- Fig. 22 und 23: perspektivische Darstellungen des Druckstücks,
- Fig. 24: eine Seitenansicht des Druckstücks,
- Fig. 25: eine Ansicht des Druckstücks in Richtung des Pfeils XXV in Fig. 24,
- Fig. 26: eine Seitenansicht des Mitnehmers,
- Fig. 27: einen Schnitt entlang der Linie XXVII-XXVII in Fig. 26,
- Fig. 28: einen Schnitt entlang der Linie XXVIII-XXVIII in Fig. 26,
- Fig. 29 und 30: perspektivische Darstellungen des Schiebers,
- Fig. 31: eine Seitenansicht des Schiebers,
- Fig. 32: eine Ansicht des Schiebers in Richtung des Pfeils XXXII in Fig. 31,
- Fig. 33: eine Seitenansicht des oberen Gehäuseteils des Injektionsgerätes,
- Fig. 34: einen Schnitt entlang der Linie XXXIV-XXXIV in Fig. 33,
- Fig. 35: einen Schnitt entlang der Linie XXXV-XXXV in Fig. 34,
- Fig. 36: einen Schnitt entlang der Linie XXXVI-XXXVI in Fig. 33,
- Fig. 37: einen Schnitt entlang der Linie XXXVII-XXXVII in Fig. 33,
- Fig. 38 und 39: perspektivische Darstellungen des Gewindeteils des Injektionsgeräts,
- Fig. 40: eine Ansicht auf das Gewindeteil von oben,
- Fig. 41: eine Seitenansicht des Gewindeteils,
- Fig. 42: einen Schnitt durch das Gewindeteil entlang der Linie XLII-XLII in Fig. 41,
- Fig. 43: eine Seitenansicht der Feder des Injektionsgerätes,
- Fig. 44 und 45: perspektivische Darstellungen der Kolbenführung,
- Fig. 46: eine Seitenansicht der Kolbenführung,
- Fig. 47: eine Ansicht der Kolbenführung in Richtung des Pfeils XLVII in Fig. 46,
- Fig. 48: eine Seitenansicht des Zustellteils,
- Fig. 49: einen Schnitt entlang der Linie XLIX-XLIX in Fig. 48,
- Fig. 50: einen Schnitt entlang der Linie L-L in Fig. 48,
- Fig. 51: einen Schnitt entlang der Linie LI-LI in Fig. 48,
- Fig. 52: Einstellteil und Rastteil eines Ausführungsbeispiels des Injektionsgerätes in perspektivischer, auseinandergezogener Darstellung,
- Fig. 53: die Anordnung aus Fig. 52 in Seitenansicht,
- Fig. 54: einen Schnitt entlang der Linie LIV-LIV in Fig. 53,
- Fig. 55: einen Schnitt entlang der Linie LV-LV in Fig. 53,
- Fig. 56: eine perspektivische Darstellung des Rastteils aus Fig. 52,
- Fig. 57: eine Draufsicht auf das Rastteil aus Fig. 56,
- Fig. 58: eine Seitenansicht des Rastteils aus Fig. 56,
- Fig. 59: eine Seitenansicht des Einstellteils aus Fig. 52,
- Fig. 60: eine Ansicht des Einstellteils in Richtung des Pfeils LX in Fig. 59,
- Fig. 61: einen Schnitt entlang der Linie LXI-LXI in Fig. 59,
- Fig. 62: eine Seitenansicht eines Ausführungsbeispiels eines Injektionsgerätes,
- Fig. 63: einen Schnitt entlang der Linie LXIII-LXIII in Fig. 62,
- Fig. 64: das Injektionsgerät aus Fig. 62 nach dem Einstellen der Maximaldosis,
- Fig. 65: einen Schnitt entlang der Linie LXV-LXV in Fig. 64,
- Fig. 66: das Injektionsgerät aus Fig. 64 nach dem Verschieben des Betätigungsknopfes in proximale Richtung,
- Fig. 67: einen Schnitt entlang der Linie LXVII-LXVII in Fig. 66,
- Fig. 68: Einstellteil, Rastteil und Feder des Injektionsgerätes aus Fig. 64 in Seitenansicht,
- Fig. 69: einen Schnitt entlang der Linie LXIX-LXIX in Fig. 68,
- Fig. 70: die Anordnung aus Fig. 68 nach dem Verschieben des Betätigungsknopfes in proximale Richtung,
- Fig. 71: einen Schnitt entlang der Linie LXXI-LXXI in Fig. 70,
- Fig. 72: eine perspektivische Darstellung des Einstellteils des Injektionsgeräts aus Fig. 62,
- Fig. 73: eine Seitenansicht des Einstellteils aus Fig. 72,
- Fig. 74: einen Schnitt entlang der Linie LXXIV-LXXIV in Fig. 73,
- Fig. 75: einen Schnitt entlang der Linie LXXV-LXXV in Fig. 73,
- Fig. 76: einen Schnitt entlang der Linie LXXVI-LXXVI in Fig. 74,
- Fig. 77 und 78: perspektivische Darstellungen des Rastteils des Injektionsgerätes aus Fig. 62,
- Fig. 79: eine Seitenansicht des Rastteils aus Fig. 77,
- Fig. 80: einen Schnitt entlang der Linie LXXX-LXXX in Fig. 79,
- Fig. 81: einen Schnitt entlang der Linie LXXXI-LXXXI in Fig. 79,
- Fig. 82: einen Schnitt entlang der Linie LXXXII-LXXXII in Fig. 79,
- Fig. 83: das Kupplungsteil des Injektionsgeräts aus Fig. 62 in perspektivischer Darstellung,
- Fig. 84 und 85: Seitenansichten des Kupplungsteils aus Fig. 83,
- Fig. 86: eine Seitenansicht des Mitnehmers des Injektionsgerätes aus Fig. 62,
- Fig. 87: einen Schnitt entlang der Linie LXXXVII-LXXXVII in Fig. 86,
- Fig. 88: eine Ansicht des Mitnehmers in Richtung des Pfeils LXXXVIII in Fig. 87,
- Fig. 89: einen Schnitt entlang der Linie LXXXIX-LXXXIX in Fig. 86,
- Fig. 90 und 91: perspektivische Darstellungen der Kolbenführung des Injektionsgeräts aus Fig. 62,
- Fig. 92: eine Seitenansicht der Kolbenführung aus Fig. 90,
- Fig. 93: eine Ansicht der Kolbenführung in Richtung des Pfeils XCIII in Fig. 92,
- Fig. 94: eine Seitenansicht des Zustellteils des Injektionsgeräts aus Fig. 62,
- Fig. 95: einen Schnitt entlang der Linie XCV-XCV in Fig. 94,
- Fig. 96: einen Schnitt entlang der Linie XCVI-XCVI in Fig. 94,
- Fig. 97: eine Seitenansicht eines Ausführungsbeispiels eines Injektionsgerätes,
- Fig. 98: einen Schnitt entlang der Linie XCVIII-XCVIII in Fig. 101,
- Fig. 99 und 100: perspektivische Darstellungen eines Rastteils des Injektionsgeräts aus Fig. 97,
- Fig. 101: eine Seitenansicht des Rastteils aus Fig. 99,
- Fig. 102: einen Schnitt entlang der Linie CII-CII in Fig. 101,
- Fig. 103: einen Schnitt entlang der Linie CIII-CIII in Fig. 101,
- Fig. 104: eine perspektivische Darstellung eines Einstellteils des Injektionsgeräts aus Fig. 97,
- Fig. 105: eine Seitenansicht des Einstellteils aus Fig. 104,
- Fig. 106: eine Schnitt entlang der Linie CVI-CVI in Fig. 105.

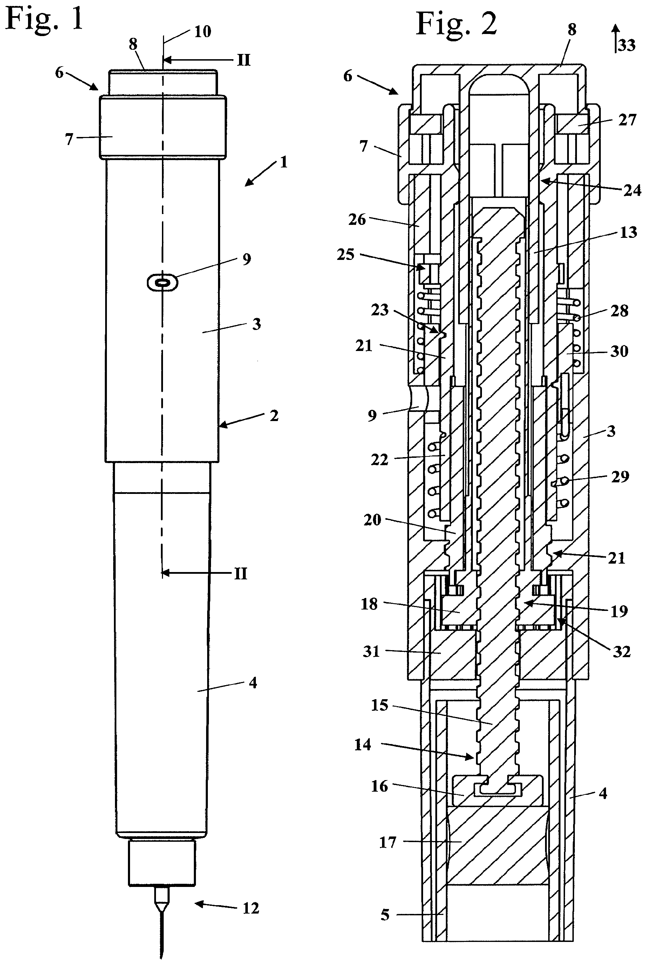

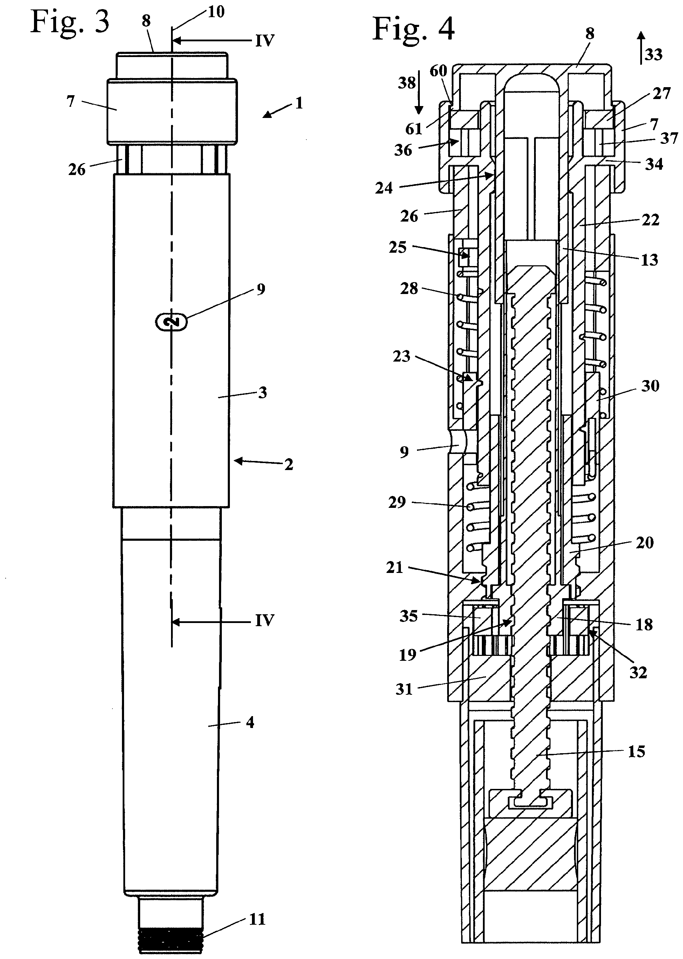

Die Figuren 1 und 2 zeigen ein Injektionsgerät 1, das ein Gehäuse 2 besitzt. Das Gehäuse 2 umfasst ein oberes Gehäuseteil 3 und unteres Gehäuseteil 4. Wie Fig. 2 zeigt, ist im unteren Gehäuseteil 4 ein Behälter 5 mit Injektionsflüssigkeit angeordnet. Das Injektionsgerät 1 dient dazu, eine vorgesehene Menge an Injektionsflüssigkeit einzustellen und aus dem Behälter 5 durch die in Fig. 1 gezeigte, am proximalen Ende des Injektionsgeräts 1 gehaltene Injektionsnadel 12 auszupressen. Die Injektionsnadel 12 ist an einem in Fig. 3 gezeigten Befestigungsgewinde 11 des Injektionsgeräts 1 lösbar befestigt.

Das proximale Ende des Injektionsgerätes 1 ist das Ende, an dem die Injektionsnadel 12 angeordnet ist. Das distale Ende des Injektionsgerätes 1 ist das der Injektionsnadel 12 abgewandt liegende Ende. Mit "proximal" wird die Seite des Injektionsgerätes 1 bezeichnet, die bei einer Injektion der Einstichstelle zugewandt liegt und mit "distal" die Seite, die der Einstichstelle abgewandt liegt. Die proximale Richtung bezeichnet die Injektionsrichtung, also die Richtung zur Injektionsnadel 12 hin bzw. die Richtung, in der die Injektionsflüssigkeit aus dem Behälter 5 ausgepresst wird. Die distale Richtung bezeichnet die entgegengesetzte Richtung, also von der Injektionsnadel 12 weg.

Das Injektionsgerät 1 besitzt ein Bedienelement 6, das im Ausführungsbeispiel mehrteilig ausgebildet ist und eine Stellhülse 7 und einen Betätigungsknopf 8 besitzt. Am oberen Gehäuseteil 3 ist ein Sichtfenster 9 vorgesehen, durch das eine Skala sichtbar ist. Die Figuren 1 und 2 zeigen das Injektionsgerät 1 in der Nullstellung, in der keine auszupressende Menge an Injektionsflüssigkeit eingestellt ist. Zum Einstellen einer auszupressenden Menge an Injektionsflüssigkeit dreht der Bediener die Stellhülse 7 um eine Längsmittelachse 10 des Injektionsgerätes 1 in einer ersten Drehrichtung, im Ausführungsbeispiel im Uhrzeigersinn. Bei der Drehung der Stellhülse 7 bewegt sich das Bedienelement 6 in distale Richtung. Zum Auspressen der eingestellten Menge an Injektionsflüssigkeit drückt der Benutzer den Betätigungsknopf 8 in proximale Richtung, bis die Stellhülse 7 in ihrer in Fig. 1 gezeigten Ausgangsposition angelangt ist.

Fig. 2 zeigt den Aufbau des Injektionsgeräts 1 im Einzelnen. Der Betätigungsknopf 8 ist fest mit einem Mitnehmer 13 verbunden. Im Ausführungsbeispiel sind der Betätigungsknopf 8 und der Mitnehmer 13 einteilig ausgebildet. Der Mitnehmer 13 ist hülsenförmig ausgebildet und ragt ins Innere des Injektionsgeräts 1. Der Mitnehmer 13 ist drehfest mit einem Zustellteil 18 verbunden. In Richtung der Längsmittelachse 10 ist der Mitnehmer 13 gegenüber dem Zustellteil 18 beweglich. Das Zustellteil 18 ist über eine erste Gewindeverbindung 19 mit der Kolbenstange 15 eines Dosierkolbens 14 verbunden. Der Dosierkolben 14 besitzt an seiner proximalen Seite eine Kolbenscheibe 16, die an einem Stopfen 17 des Behälters 5 anliegt. Zum Auspressen von Injektionsflüssigkeit wird der Dosierkolben 14 in proximale Richtung verschoben und bewegt dadurch den Stopfen 17 in proximale Richtung, wodurch Injektionsflüssigkeit aus dem Behälter 5 ausgepresst wird.

Die Kolbenstange 15 ist über eine zwischen dem oberen Gehäuseteil 3 und dem unteren Gehäuseteil 4 gehaltene Kolbenführung 31 drehfest im Gehäuse 2 gehalten. Die Kolbenführung 31 ist dabei drehfest mit dem oberen Gehäuseteil 3 verbunden. Der Mitnehmer 13 ist über eine Kupplung 24 mit einem Einstellteil 22 verbunden. Das Einstellteil 22 ist fest mit der Stellhülse 7 verbunden, im Ausführungsbeispiel einteilig mit dieser ausgeführt. In der in Fig. 1 und 2 gezeigten Stellung des Injektionsgeräts 1 ist die Kupplung 24 geschlossen und verbindet den Mitnehmer 13 drehfest mit dem Einstellteil 22. Am Außenumfang des Zustellteils 18 ist ein Schieber 20 angeordnet, der über eine zweite Gewindeverbindung 21 im Gehäuse 2 gehalten ist. Das Einstellteil 22 ist im Ausführungsbeispiel am Außenumfang des Schiebers 20 angeordnet. Der Schieber 20 und das Einstellteil 22 sind drehfest miteinander verbunden. Im Gehäuse 2 ist ein Gewindeteil 30 fixiert. Das Gewindeteil 30 ist über eine dritte Gewindeverbindung 23 mit dem Einstellteil 22 verbunden. Das Gewindeteil 30 könnte auch als Teil des Gehäuses 2 ausgeführt sein, da das Gewindeteil 30 drehfest und axial fest im Gehäuse 2 gehalten ist. Durch die mehrteilige Ausbildung ergibt sich eine vereinfachte Herstellung.

Zwischen dem Gewindeteil 30 bzw. dem Gehäuse 2 und dem Schieber 20 wirkt eine Feder 29, die vorteilhaft als Torsionsfeder ausgebildet ist. Die Feder 29 spannt den Schieber 20 in Richtung auf die in den Figuren 1 und 2 gezeigte Nullstellung vor. Beim Einstellen einer auszupressenden Menge an Injektionsflüssigkeit wird die Feder 29 gespannt. Beim Auspressen von Injektionsflüssigkeit unterstützt die Feder 29 den Benutzer und verringert die zum Auspressen der Injektionsflüssigkeit benötigte Kraft.

Im oberen Gehäuseteil 3 ist ein Rastteil 26 drehfest und axial verschiebbar angeordnet. Das Injektionsgerät 1 besitzt eine erste Rasteinrichtung 25, die zwischen dem Rastteil 26 und dem Einstellteil 22 wirkt. In axialer Richtung zwischen dem Betätigungsknopf 8 und dem Rastteil 26 ist ein Druckstück 27 angeordnet, dessen Funktion im Folgenden noch näher erläutert wird. Das Rastteil 26 ist von einer Feder 28, die als Druckfeder ausgebildet ist, in Richtung auf seine distale Position vorgespannt.

Die Figuren 3 und 4 zeigen das Injektionsgerät 1 in einer Stellung, in der die Maximaldosis eingestellt ist. Im Sichtfenster 9 ist als Anzeige für die Maximaldosis eine "2" erkennbar. Zum Einstellen einer Dosis dreht der Bediener die Stellhülse 7. Über die Kupplung 24 dreht sich der Mitnehmer 13 mit dem Betätigungsknopf 8 mit, und über die drehfeste Verbindung zwischen Mitnehmer 13 und Zustellteil 18 dreht sich auch das Zustellteil 18. Aufgrund der ersten Gewindeverbindung 19 bewegt sich das Zustellteil 18 gleichzeitig in Richtung des Pfeils 33 in distale Richtung. Zwischen dem Zustellteil 18 und der Kolbenführung 31 ist eine zweite Rasteinrichtung 32 ausgebildet. Beim Einstellen einer auszupressenden Menge an Injektionsflüssigkeit sind aufgrund der zweiten Rasteinrichtung 32 schwache Klicks spür- und hörbar.

Das Einstellteil 22 bewegt sich beim Drehen der Stellhülse 7 aufgrund der dritten Gewindeverbindung 23 in Richtung des Pfeils 33 in distale Richtung. Die Steigung der dritten Gewindeverbindung 23 kann dabei deutlich größer als die der ersten Gewindeverbindung 19 sein. Am Außenumfang des Einstellteils 22 ist die Skala aufgebracht, die durch das Sichtfenster 9 sichtbar ist. Aufgrund der drehfesten Verbindung zwischen Einstellteil 22 und Schieber 20 dreht sich auch der Schieber 20 mit. Aufgrund der zweiten Gewindeverbindung 21 bewegt sich der Schieber 21 zusätzlich in Richtung des Pfeils 33 in distale Richtung. Die Steigung der zweiten Gewindeverbindung 21 ist dabei vorteilhaft mindestens so groß wie die Steigung der ersten Gewindeverbindung 19, so dass die Bewegung des Zustellteils 18 in distale Richtung durch den Schieber 20 nicht behindert wird.

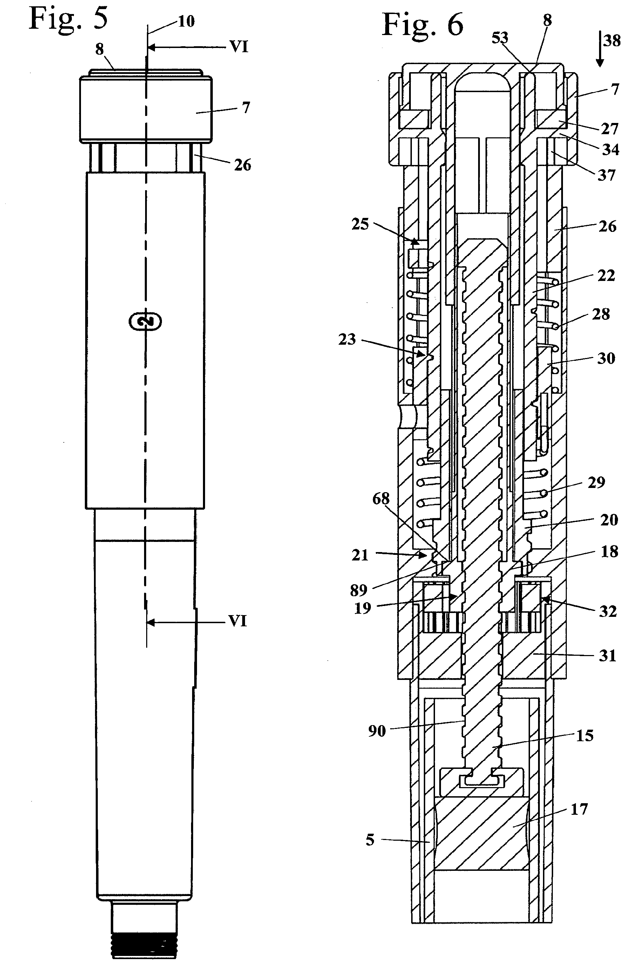

Nach dem Einstellen der auszupressenden Menge an Injektionsflüssigkeit muss der Bediener den Betätigungsknopf 8 in Richtung des Pfeils 38, also in proximale Richtung, drücken. In der Stellhülse 7 ist eine Aufnahme 36 ausgebildet, in der das Druckstück 27 angeordnet ist. Das Druckstück 27 besitzt Druckstege 37, die in proximale Richtung zum Rastteil 26 ragen. Die Stellhülse 7 ist über einen Ringsteg 34 mit dem hülsenförmigen Grundkörper des Einstellteils 22 verbunden. Beim Drücken des Betätigungsknopfes 8 werden die Druckstege 37 durch in Fig. 4 nicht gezeigte Öffnungen im Ringsteg 34 gedrückt und schieben dadurch das Rastteil 26 in proximale Richtung. Dadurch gelangen die Rastelemente der ersten Rasteinrichtung 25 außer Eingriff. Diese Stellung ist in den Figuren 5 und 6 gezeigt. Dadurch, dass die Rasteinrichtung 25 ein Zurückdrehen der Stellhülse 7 nicht mehr verhindert, kann der Bediener den Betätigungsknopf 8 zusammen mit der Stellhülse 7 weiter in Richtung des Pfeils 38 in proximale Richtung bewegen. Dadurch bewegt sich die Stellhülse 7 in proximale Richtung zurück in ihre Ausgangslage und dreht sich dabei zusammen mit dem Einstellteil 22 aufgrund der dritten Gewindeverbindung 23. Der Schieber 20 dreht sich ebenfalls zurück und bewegt sich in proximale Richtung in seine Ausgangslage. Wie Fig. 6 zeigt, besitzt der Schieber 20 einen Absatz 68, der an einem Absatz 89 des Zustellteils 18 anliegt. Der Schieber 20 nimmt bei seiner Bewegung in proximale Richtung über die Absätze 68 und 89 das Zustellteil 18 mit und verschiebt es dadurch ebenfalls in proximale Richtung. Das Zustellteil 18 ist drehfest mit dem Betätigungsknopf 8 verbunden und kann sich nicht drehen, sondern wird, geführt von der zweiten Rasteinrichtung 32, in Längsrichtung des Injektionsgeräts 1 bewegt. Da die Kolbenstange 15 drehfest im Gehäuse 2 gehalten ist, wird die Kolbenstange 15 über die erste Gewindeverbindung 19 mitgenommen und bewegt den Stopfen 17 in proximale Richtung. Dadurch wird die eingestellte Menge an Injektionsflüssigkeit aus dem Behälter 5 ausgepresst. In Fig. 6 ist auch ein an der Kolbenstange 15 vorgesehenes Außengewinde 90 sichtbar, das Teil der ersten Gewindeverbindung 19 ist.

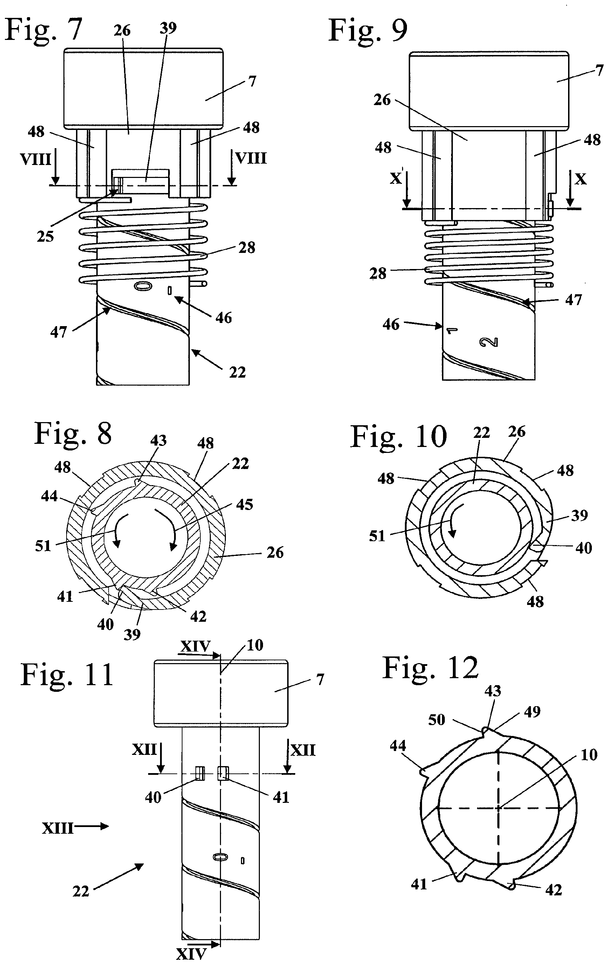

Die Figuren 7 bis 10 zeigen die Gestaltung der ersten Rasteinrichtung 25 im Einzelnen. Die erste Rasteinrichtung 25 umfasst einen Rastarm 39 am Rastteil 26, an dem das in Fig. 8 gezeigt Rastelement 40 ausgebildet ist. In der in den Figuren 7 und 8 gezeigten axialen Relativposition von Rastteil 26 und Einstellteil 22 wirkt das Rastelement 40 des Rastteils 26 mit Rastelementen 41, 42, 43 und 44 am Einstellteil 22 zusammen und bildet mit diesen die erste Rasteinrichtung 25. Die Figuren 7 und 8 zeigen die Anordnung in Nullstellung bei unbetätigtem Betätigungsknopf 8. Diese Stellung des Injektionsgerätes 1 ist in den Figuren 1 und 2 ebenfalls gezeigt. Fig. 7 zeigt auch die am Außenumfang des Einstellteils 22 angeordnete Skala 46 sowie das Außengewinde 47, das Teil der dritten Gewindeverbindung 23 ist. Das Rastteil 26 besitzt Längsnuten 48, mit denen das Rastteil 26 drehfest aber axial verschiebbar im oberen Gehäuseteil 3 (Fig. 2) geführt ist.

Beim Einstellen einer einzustellenden Menge an Injektionsflüssigkeit wird das Einstellteil 22 in der in Fig. 8 gezeigten ersten Drehrichtung 45 gedreht. Fig. 8 zeigt die Anordnung in Nullstellung. Rastet das Rastelement 40 am Rastelement 42 des Einstellteils 22 ein, so ist die Priming-Stellung erreicht. Bei weiterem Drehen des Einstellteils 22 wird eine weitere Raststellung, die einer ersten Dosis zugeordnet ist, am Rastelement 43 sowie eine weitere Raststellung, die einer zweiten Dosis, die gleichzeitig die Maximaldosis ist, zugeordnet ist, am Rastelement 44 erreicht. Wie Fig. 8 zeigt, besitzen die Rastelemente 41, 42, 43 und 44 unterschiedliche Abstände in Umfangsrichtung zueinander. Die Rastelemente 40 bis 44 sind so ausgebildet, dass das Einstellteil 22 bei aktiver Rasteinrichtung 25, also bei der in den Figuren 7 und 8 gezeigten relativen axialen Lage von Einstellteil 22 und Rastteil 26, nur in der ersten Drehrichtung 45 gedreht werden kann. Nach Erreichen einer Raststellung ist ein Zurückstellen nicht mehr möglich. Wird die Stellhülse 7 losgelassen, während sich das Einstellteil 22 zwischen zwei Raststellungen befindet, so dreht die Feder 29 den Schieber 20 und damit auch das drehfest mit dem Schieber 20 verbundene Einstellteil 22 zurück, bis die nächst niedrige Raststellung erreicht ist. Dadurch ist das Auspressen einer Menge an Injektionsflüssigkeit, der keine Raststellung der ersten Rasteinrichtung 25 zugeordnet ist, vermieden.

Die Figuren 9 und 10 zeigen die Anordnung nach dem Drücken des Betätigungsknopfes 8, wie sie in den Figuren 5 und 6 gezeigt ist. Durch Drücken des Betätigungsknopfes 8 in proximale Richtung wird das Rastteil 26 in proximale Richtung verschoben. Dadurch gelangt der Rastarm 39 mit dem Rastelement 40 außer Eingriff mit den Rastelementen 41 bis 44 am Einstellteil 22. Das Einstellteil 22 kann sich in einer der ersten Drehrichtung 45 entgegen gerichteten zweiten Drehrichtung 51 zurück in seine Ausgangsposition drehen. Dabei wird die eingestellte Menge an Injektionsflüssigkeit ausgepresst.

Die Figuren 11 bis 16 zeigen die Gestaltung des Einstellteils 22 im Einzelnen. Wie Fig. 12 zeigt, besitzt jedes Rastelement eine Führungsflanke 49, die vergleichsweise flach zum Außenumfang des Einstellteils 22 verläuft, sowie eine Rastflanke 50, die steil, im Ausführungsbeispiel näherungsweise senkrecht, zum Außenumfang des Einstellteils 22 verläuft. Der unterschiedliche Neigungswinkel von Führungsflanke 49 und Rastflanke 50 stellt sicher, dass die Raststellungen leicht erreicht, das Einstellteil 22 aber nicht aus einer Raststellung zu einer niedrigeren Dosis zurückgestellt werden kann. Es kann jedoch auch vorgesehen sein, die Rastelemente 40 bis 44 so auszubilden, dass die Rastflanke 50 durch Drehen des Einstellteils 22 in der zweiten Drehrichtung 51 überwunden und das Einstellteil 22 zurückgestellt werden kann.

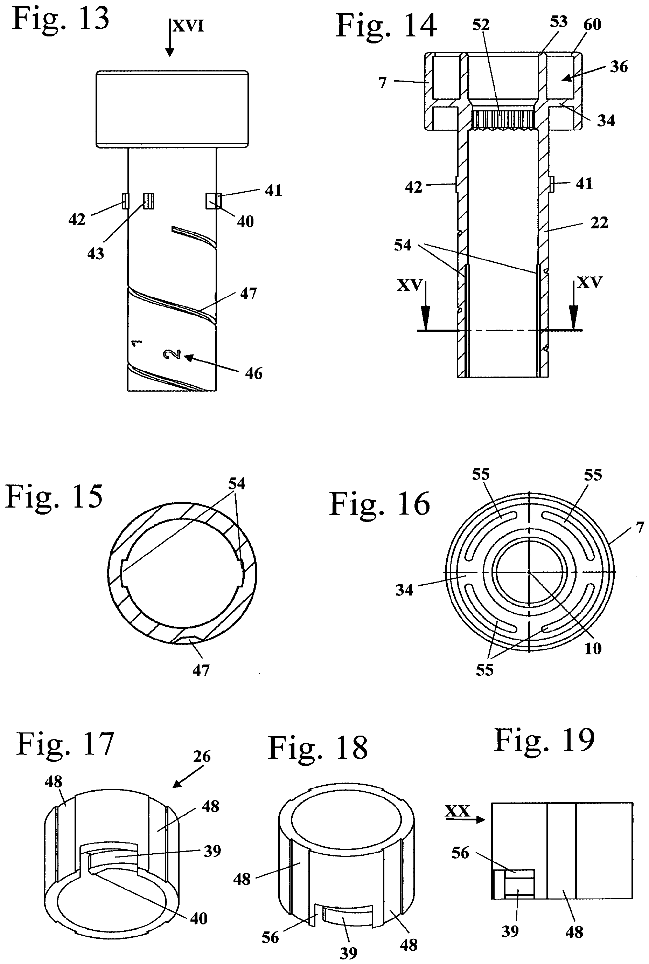

Wie Fig. 14 zeigt, besitzt das Einstellteil 22 an seiner Innenseite eine Verzahnung 52. Die Verzahnung 52 ist Teil der Kupplung 24 und im Ausführungsbeispiel etwa auf der Höhe der Stellhülse 7 angeordnet. Am distalen Ende des Einstellteils 22 ist ein Anschlag 53 für den Betätigungsknopf 8 ausgebildet. Wie Fig. 5 zeigt, liegt der Betätigungsknopf 8 in vollständig in die Stellhülse7 gedrücktem Zustand am Anschlag 53 an. In seinem proximalen Bereich besitzt das Einstellteil 22 am Innenumfang zwei Längsnuten 54, die einander gegenüberliegend angeordnet sind und zur drehfesten Verbindung des Einstellteils 22 mit dem Schieber 20 dienen. An der distalen Seite der Stellhülse 7 ist ein Halterand 60 vorgesehen, der den Betätigungsknopf 8 hält.

Fig. 16 zeigt den Ringsteg 34. Wie Fig. 16 zeigt, sind am Ringsteg 34 vier Öffnungen 55 ausgebildet, durch die die Druckstege 37 (Fig. 6) des Druckstücks 27 ragen. Die Öffnungen 55 sind bogenförmig um die Längsmittelachse 10 ausgebildet.

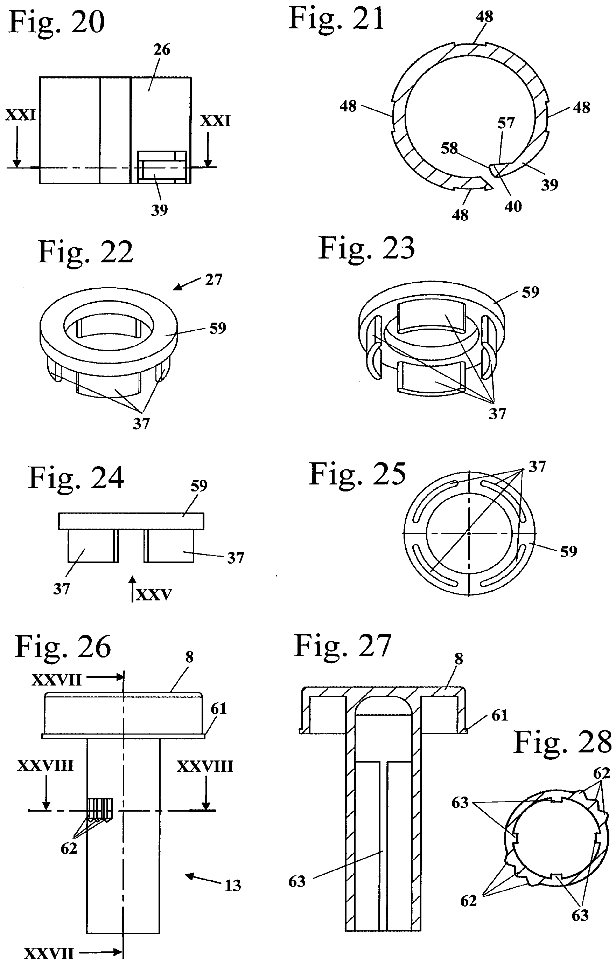

In den Figuren 17 bis 21 ist das Rastteil 26 im Einzelnen gezeigt. Wie die Figuren 17 bis 19 zeigen, ist der Rastarm 39 benachbart zum proximalen Ende des Rastteils 26 in einer Aussparung 56 angeordnet. Wie Fig. 21 zeigt, besitzt das Rastelement 40 eine Führungsflanke 57, die geringfügig zur Umfangsrichtung geneigt ist, sowie eine steil ausgerichtete Rastflanke 58, die zum Einrasten hinter einer Rastflanke 50 eines der Rastelemente 41 bis 44 ausgebildet ist. Das Rastelement 39 ist radial nach innen federnd ausgebildet, und zwar aufgrund der Eigenelastizität des Materials. Vorteilhaft besteht das Rastteil 26 aus Kunststoff. Wie Fig. 21 auch zeigt, sind gleichmäßig am Außenumfang verteilt vier Längsnuten 48 zur drehfesten Verbindung mit dem oberen Gehäuseteil 3 vorgesehen.

Die Figuren 22 bis 25 zeigen das Druckstück 27 im Einzelnen. Das Druckstück 27 besitzt einen Ringabschnitt 59, an dem der Betätigungsknopf 8 an der distalen Seite anliegt. In proximale Richtung ragen vom Ringabschnitt 59 vier Druckstege 37, die jeweils kreisbogenförmig ausgebildet sind. Die Druckstege 37 ragen in unbetätigtem Zustand des Betätigungsknopfes 8 durch die Öffnungen 55 des Ringstegs 34 (Fig. 16), jedoch nicht oder nur geringfügig durch diese hindurch.

Die Figuren 26 bis 28 zeigen den Mitnehmer 13 mit dem daran angeformten Betätigungsknopf 8 im Einzelnen. Wie die Figuren 26 und 27 zeigen, weist der Betätigungsknopf 8 einen nach außen ragenden Halterand 61 auf. Der Halterand 61 liegt in unbetätigtem Zustand des Betätigungsknopfes 8 am Halterand 60 des Einstellteils 22 an und sichert dadurch den Betätigungsknopf 8 in distale Richtung, wie Fig. 4 zeigt. Wie die Figuren 26 und 28 zeigen, besitzt der Mitnehmer 13 an seinem Außenumfang Zähne 62. Im Ausführungsbeispiel sind zwei Gruppen von je drei Zähnen 62 einander gegenüberliegend am Außenumfang des Mitnehmers 13 angeordnet. Auch eine andere Anzahl von Zähnen 62 kann vorteilhaft sein. Die Zähne 62 wirken mit der Verzahnung 52 des Einstellteils 22 zusammen und bilden mit dieser die Kupplung 24. Sind die Zähne 62 in der Verzahnung 52 angeordnet, so ist die Kupplung 24 geschlossen. Dies ist in den Figuren 1 bis 4 gezeigt. Wird der Betätigungsknopf 8 gedrückt, so gelangen die Zähne 62 außer Eingriff mit der Verzahnung 52. Diese Stellung des Injektionsgeräts 2 ist in den Figuren 5 und 6 gezeigt. In dieser Position ist das Einstellteil 22 gegenüber dem Mitnehmer 13 drehbar.

Wie Figuren 27 und 28 zeigen, besitzt der Mitnehmer 13 in seinem Innenumfang 4 Längsstege 63. Die Längsstege 63 dienen zur drehfesten Verbindung mit dem Zustellteil 18.

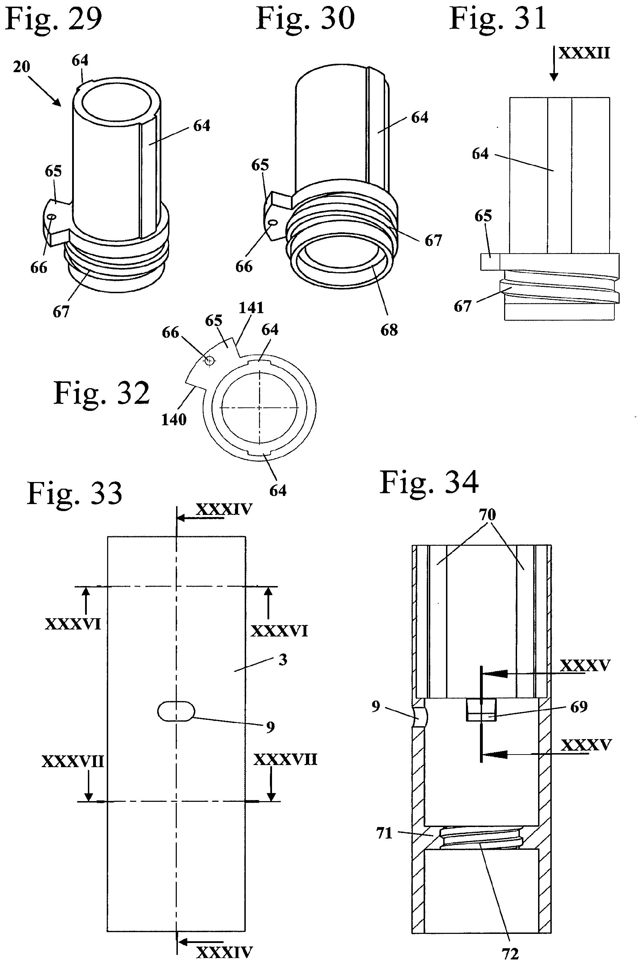

Die Figuren 29 bis 32 zeigen den Schieber 20 im Einzelnen. An seinem proximalen Ende trägt der Schieber 20 ein Außengewinde 67. In seinem distalen Bereich weist der Schieber 20 an seinem Außenumfang zwei einander gegenüberliegend angeordnete Längsstege 64 auf, die zur drehfesten Verbindung mit dem Einstellteil 22 dienen. Hierzu ragen die Längsstege 64 in die Längsnuten 54 des Einstellteils 22. An der distalen Seite des Außengewindes 67 weist der Schieber 20 einen nach außen auskragenden Arm 65 auf. Der Arm 65 besitzt eine Öffnung 66, in der die Feder 29 eingehängt ist. Wie Fig. 30 zeigt, besitzt der Schieber 20 benachbart zu seinem proximalen Ende an seiner Innenseite den Absatz 68. Wie Fig. 32 zeigt, ist am Arm 65 ein erster Anschlag 140 zur Festlegung der Nullstellung und ein zweiter Anschlag 141 zur Festlegung der maximal einstellbaren Dosis vorgesehen. Die Funktion der Anschläge 140 und 141 wird im Folgenden noch näher beschrieben.

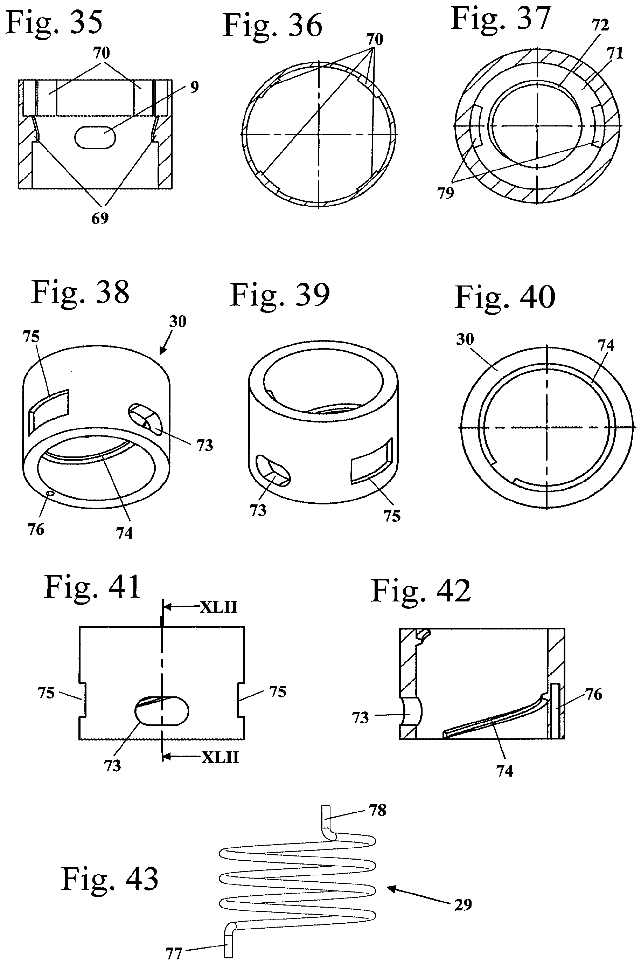

Die Figuren 33 bis 37 zeigen den Aufbau des oberen Gehäuseteils 3 im Einzelnen. Das obere Gehäuseteil 3 besitzt etwa auf der Höhe des Sichtfensters 6 zwei einander gegenüberliegend angeordnete Rasten 69. In seinem distalen Bereich weist das obere Gehäuseteil 3 vier Längsstege 70 auf, die in die Längsnuten 48 des Rastteils 26 ragen und das Rastteil 26 drehfest und axial verschiebbar im oberen Gehäuseteil 3 halten. Das obere Gehäuseteil 3 besitzt einen nach innen ragenden Ringsteg 71, an dem ein Innengewinde 72 ausgebildet ist. Das Innengewinde 72 wirkt mit dem Außengewinde 67 des Schiebers 20 zusammen und bildet mit diesem die zweite Gewindeverbindung 21. Wie Fig. 37 zeigt, besitzt der Ringsteg 71 zwei Öffnungen 79, die zur drehfesten Fixierung der Kolbenführung 31 dienen.

Die Figuren 38 bis 42 zeigen das Gewindeteil 30. Das Gewindeteil 30 besitzt zwei einander gegenüberliegend angeordnete Rastöffnungen 75 zur Aufnahme der Rasten 69 des oberen Gehäuseteils 3. Die Rasten 69 fixieren das Gewindeteil 30 im oberen Gehäuseteil 3. Das Gewindeteil 30 besitzt ein Innengewinde 74, das mit dem Außengewinde 47 des Einstellteils 22 zusammenwirkt und mit diesem die dritte Gewindeverbindung 23 bildet. Das Gewindeteil 30 besitzt außerdem ein Sichtfenster 73, das in Überdeckung zum Sichtfenster 9 angeordnet ist und durch das die Skala 46 auf dem Einstellteil 22 für den Bediener sichtbar ist. Wie die Figuren 38 und 42 zeigen, besitzt das Gewindeteil 30 eine Aufnahme 76 für ein Ende der Feder 29.

Fig. 43 zeigt die Feder 29. Die Feder 29 besitzt ein erstes Ende 77, das in der Öffnung 66 des Schiebers 20 anzuordnen ist, sowie ein zweites Ende 78, das im Betrieb in die Aufnahme 76 des Gewindeteils 30 ragt und dadurch das zweite Ende 78 der Feder 29 gehäusefest fixiert. Dadurch sind der Schieber 20 und das drehfest mit dem Schieber 20 verbundene Einstellteil 20 in Richtung auf die Nullstellung des Injektionsgeräts 1, also in der zweiten Drehrichtung 51, vorgespannt.

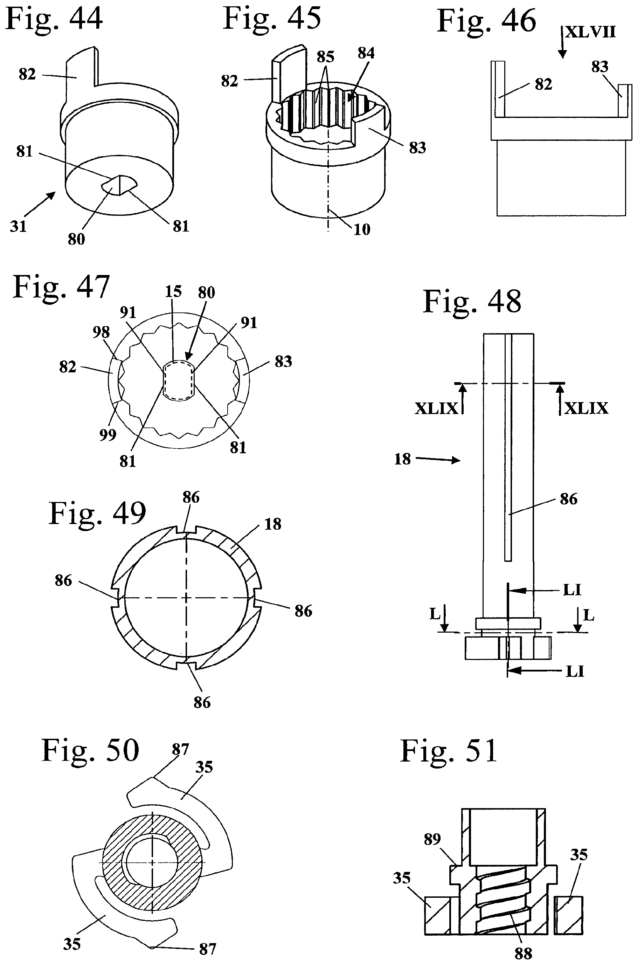

Die Figuren 44 bis 47 zeigen die Kolbenführung 31 im Einzelnen. Die Kolbenführung 31 besitzt zwei Arme 82 und 83, die durch die in Fig. 37 gezeigten Öffnungen 79 des oberen Gehäuseteils 3 ragen. Der Arm 82 ist länger als der Arm 83 ausgebildet. Der Arm 82 besitzt einen ersten Anschlag 98 und einen zweiten Anschlag 99. Der erste Anschlag 98 wirkt im Betrieb mit dem in Fig. 32 gezeigten ersten Anschlag 140 des Schiebers 20 zusammen und legt mit diesem die Nullstellung des Injektionsgeräts fest. Der zweite Anschlag 99 wirkt mit dem zweiten Anschlag 141 des Schiebers 20 zur Festlegung der Maximalstellung zusammen.

Wie Fig. 45 und Fig. 47 zeigen, besitzt die Kolbenführung 31 eine Raststruktur 84, die durch eine Vielzahl von parallel zur Längsmittelachse 10 verlaufenden Längsstegen 85 gebildet ist. Die Kolbenführung 31 besitzt außerdem eine Öffnung 80, die zwei einander gegenüberliegend angeordnete Abflachungen 81 besitzt. Wie in Fig. 47 schematisch eingezeichnet ist, besitzt die Kolbenstange 15 entsprechende Abflachungen 91. Über die Abflachungen 81 und 91 ist die Kolbenstange 15 drehfest in der Kolbenführung 31 gehalten, die ihrerseits über die Arme 82 und 83 drehfest mit dem oberen Gehäuseteil 3 verbunden ist. Dadurch kann sich die Kolbenstange 15 gegenüber dem Gehäuse 2 nicht drehen.

Die Figuren 48 bis 51 zeigen das Zustellteil 18 im Einzelnen. Das Zustellteil 18 besitzt an seinem proximalen Ende zwei Rastarme 35, an deren Ende jeweils ein nach außen ragendes Rastelement 87 angeordnet ist. Die Rastelemente 87 wirken mit der Raststruktur 84 der Kolbenführung 31 zusammen und bilden mit dieser die zweite Rasteinrichtung 32. Da sich das Zustellteil 18 beim Einstellen einer Dosis gegenüber dem Gehäuse 3 dreht und beim Auspressen der Dosis an den Längsstegen 85 in Richtung der Längsmittelachse 10 geführt ist, ändert sich die Relativposition der Rastelemente 87 zum Gehäuse 2 nach jedem Injektionsvorgang. Die Raststruktur 84 ist so ausgebildet, dass die hier definierten Rastpositionen einen Teiler für alle mit der Rasteinrichtung 25 einstellbaren Rastpositionen bilden. Die Raststellungen der zweiten Rasteinrichtung 32 sind außerdem deutlich schwächer spür- und hörbar als die der ersten Rasteinrichtung 25, so dass für den Bediener deutlich erkennbar ist, wann eine zulässige Dosis eingestellt ist. Die Längsstege 85 der Raststruktur 84 sind symmetrisch ausgebildet, so dass die zweite Rasteinrichtung 32 zurückgestellt werden kann. Die zweite Rasteinrichtung 32 ist so ausgelegt, dass das in der Feder 29 gespeicherte Drehmoment ausreichend ist, um die Kraft der zweiten Rasteinrichtung 32 zu überwinden und das Einstellteil 22 zurück zur nächst niedrigen zulässigen Dosis zu stellen, wenn eine nicht zulässige Dosis eingestellt wurde.

Wie Fig. 49 zeigt, besitzt das Zustellteil 18 in seinem distalen Bereich vier gleichmäßig am Umfang verteilte Längsnuten 86. In die Längsnuten 86 ragen die Längsstege 63 des Mitnehmers 13. Dadurch ist der Mitnehmer 13 drehfest mit dem Zustellteil 18 verbunden. An seinem proximalen Ende weist das Zustellteil 18 ein Innengewinde 88 auf, das mit dem Außengewinde 90 der Kolbenstange 15 zusammenwirkt und mit diesem die erste Gewindeverbindung 19 bildet. Fig. 51 zeigt auch den Absatz 89 des Zustellteils 18, an dem der Schieber 20 anliegt.

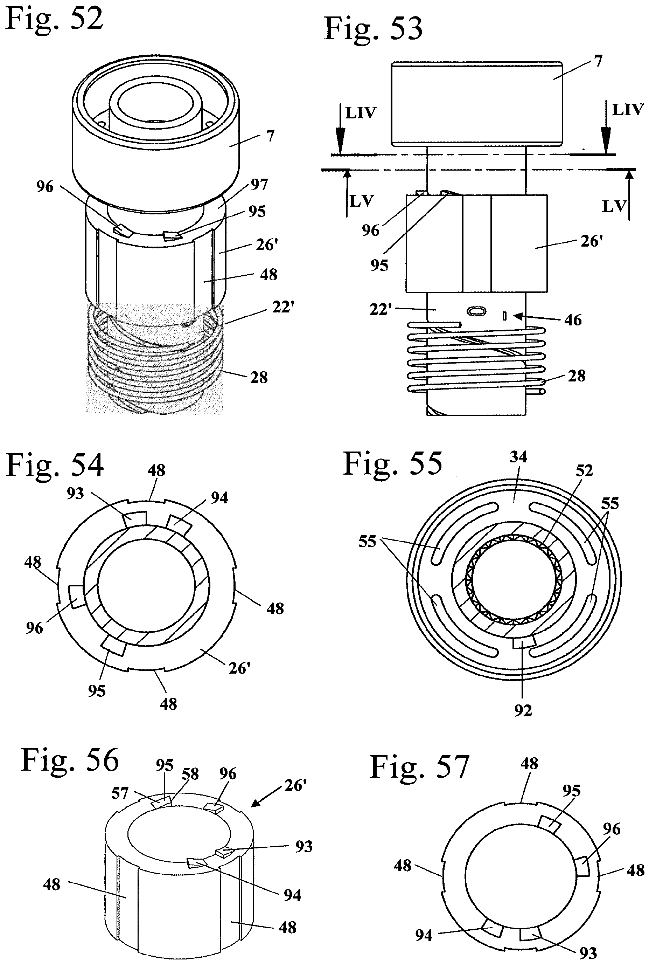

Die Figuren 52 bis 61 zeigen ein weiteres Ausführungsbeispiel für Einstellteil und Rastteil des Injektionsgeräts 1. Gleiche Elemente sind mit gleichen Bezugszeichen versehen und einander entsprechende Elemente mit einem Strich gekennzeichnet.

Die Figuren 52 und 53 zeigen ein Einstellteil 22' mit einem Rastteil 26'. Das Rastteil 26' ist in einer distalen Position angeordnet, die im Betrieb nicht erreicht werden kann, um die Elemente besser sichtbar zu machen. Das Rastteil 26' besitzt an seiner distalen Stirnseite 97 Rastelemente 93, 94, 95 und 96. Das Rastelement 93 ist der Nullstellung zugeordnet, das Rastelement 94 der Priming-Position, das Rastelement 95 einer ersten Dosis und das Rastelement 96 einer zweiten, maximal einstellbaren Dosis. Die Feder 28 spannt das Rastteil 26' in distale Richtung vor. Wie Fig. 55 zeigt, besitzt das Einstellteil 22' am Ringsteg 34 ein Rastelement 92. Die Figuren 56 bis 58 zeigen die Anordnung der Rastelemente 93 bis 96 am Rastteil 26' im Einzelnen. Wie Fig. 56 zeigt, besitzt jedes Rastelement eine Führungsflanke 57 und eine Rastflanke 58. Die Rastflanken 57 verlaufen flach, während die Rastflanke 58 steil, näherungsweise parallel zur Längsmittelachse 10 verläuft.

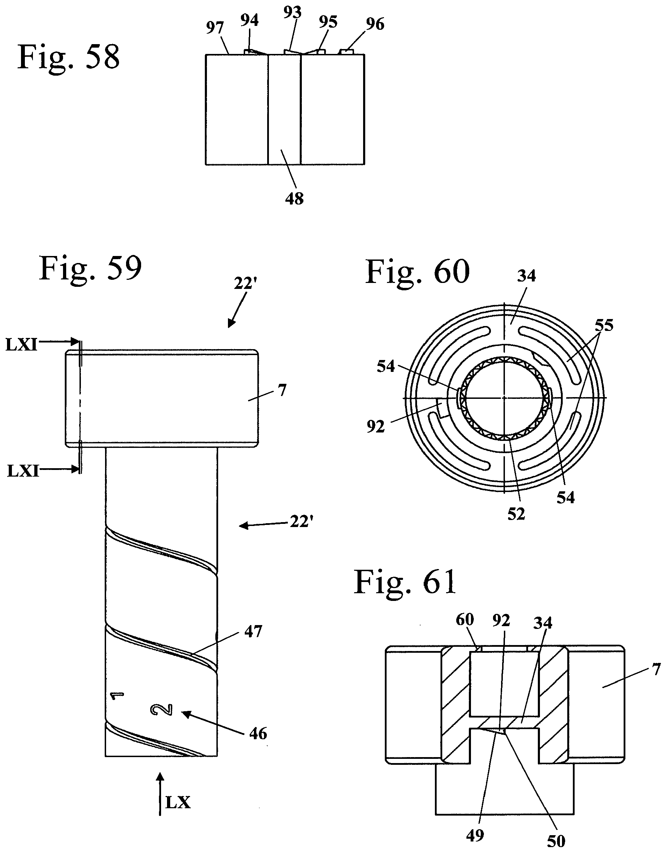

Wie Fig. 61 zeigt, besitzt das Rastelement 92 eine Führungsflanke 49 und eine Rastflanke 50. Auch die Führungsflanke 49 verläuft flach, während die Rastflanke 50 steil verläuft. Auch bei dem in den Figuren 52 bis 61 gezeigten Ausführungsbeispiel ist dadurch ein Zurückstellen einer einmal eingestellten zulässigen Menge an Injektionsflüssigkeit nicht möglich. Die in den Figuren 52 bis 61 gezeigte Rasteinrichtung ist aktiv, wenn das Rastteil 26 sich in seiner distalen Position befindet. Wird der Betätigungsknopf 8 (Fig. 1) in proximale Richtung gedrückt und über das Druckstück 27 (Fig. 2) das Rastteil 26 in proximale Richtung bewegt, so gelangen die Rastelemente 93 bis 96 außer Eingriff mit dem Rastelement 92. Das Einstellteil 22' kann sich dann in seine Ausgangslage zurückstellen. Die Rastelemente 92 bis 96 wirken in axialer Richtung, während die Rastelemente 40 bis 44 in radialer Richtung wirken.

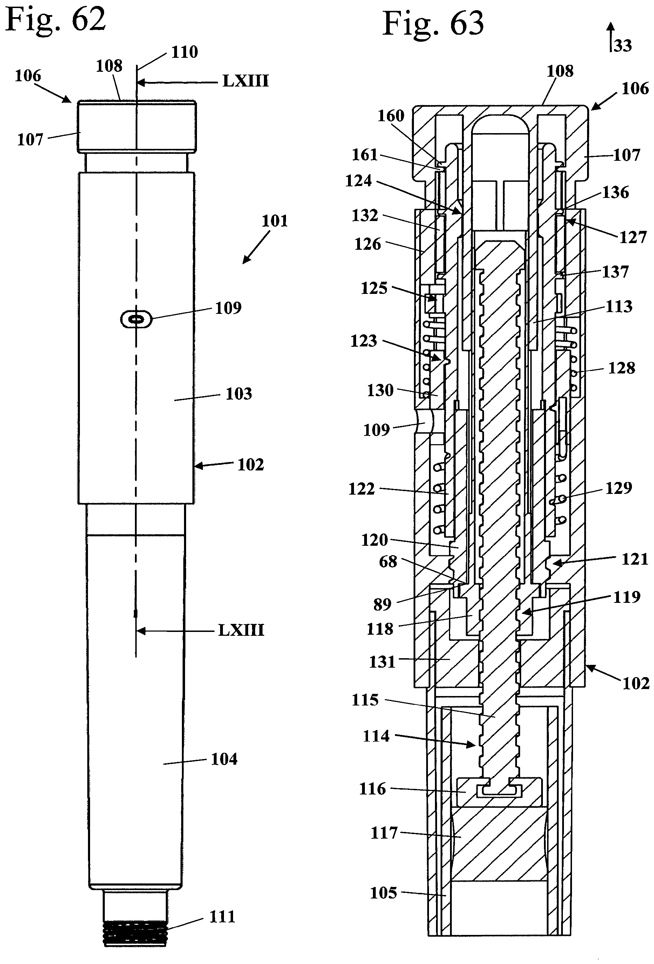

Die Figuren 62 bis 96 zeigen ein Ausführungsbeispiel eines Injektionsgeräts 101. Gleiche Bezugszeichen wie in den voran gegangenen Figuren kennzeichnen gleiche Elemente. Das Injektionsgerät 101 ist in den Figuren 62 und 63 in Nullstellung gezeigt. Das Injektionsgerät 101 besitzt ein Gehäuse 102, das ein oberes Gehäuseteil 103 und ein an der proximalen Seite des oberen Gehäuseteils 103 angeordnetes unteres Gehäuseteil 104 umfasst. Im unteren Gehäuseteil 104 ist ein Behälter 105 mit Injektionsflüssigkeit angeordnet, in dem ein Stopfen 117 angeordnet ist. Das obere Gehäuseteil 103 besitzt ein Sichtfenster 109, durch das eine Skala sichtbar ist. Am distalen Ende des oberen Gehäuseteils 103 ist ein Bedienelement 106 angeordnet, das eine Stellhülse 107 und einen Betätigungsknopf 108 umfasst, die fest miteinander verbunden sind. Wie Fig. 63 zeigt, sind Stellhülse 107 und Betätigungsknopf 108 im Ausführungsbeispiel einteilig miteinander ausgebildet. Das Bedienelement 106 ist auch einteilig mit einem Mitnehmer 113 ausgebildet. Im Bereich der Stellhülse 107 besitzt das Bedienelement 106 einen nach innen ragenden Halterand 161, der an einem Halterand 160 eines Einstellteils 122 anliegt. Das Bedienelement 106 ist an der distalen Seite eines Rastteils 126 angeordnet, das von einer Feder 128 in distale Richtung vorgespannt ist. Die Feder 128 wirkt über das Rastteil 126 auch auf das Bedienelement 106. Die Halteränder 160 und 161 verhindern eine weitere Bewegung des Bedienelements 106 in distale Richtung. Das Injektionsgerät 101 besitzt eine Längsmittelachse 110. Am distalen Ende des Injektionsgerätes 101 ist ein Befestigungsgewinde 111 für eine Injektionsnadel vorgesehen.

Der Mitnehmer 113 ist mit einem Zustellteil 118 drehfest verbunden, das über eine erste Gewindeverbindung 119 mit einer Kolbenstange 115 eines Dosierkolbens 114 verbunden ist. Der Dosierkolben 114 trägt an seiner proximalen Seite eine Kolbenscheibe 116, die am Stopfen 117 des Behälters 105 anliegt. Die Kolbenstange 115 ist in einer Kolbenführung 131 drehfest gegenüber dem Gehäuse 102 gehalten. Das Zustellteil 118 besitzt den Absatz 89, an dem der Absatz 68 eines Schiebers 120 anliegt. Der Schieber 120 ist über eine zweite Gewindeverbindung 121 mit dem Gehäuse 102 verbunden. Der Schieber 120 ist drehfest mit einem Einstellteil 122 verbunden, das über eine dritte Gewindeverbindung 123 mit dem Gehäuse 103 verbunden ist. Die dritte Gewindeverbindung 123 ist zwischen einem im Gehäuse 102 fest gehaltenen Gewindeteil 130 und dem Einstellteil 122 ausgebildet. Am Außenumfang des Gewindeteils 130 ist die Feder 128 geführt. Zwischen dem Gewindeteil 130 und dem Schieber 120 wirkt eine zweite Feder 129, die als Torsionsfeder ausgebildet ist und die den Schieber 120 in Richtung auf die Nullstellung des Injektionsgeräts 101 vorspannt. Zwischen dem Rastteil 126 und dem Einstellteil 122 wirkt eine Rasteinrichtung 125.

Zwischen dem Einstellteil 122 und dem Mitnehmer 113 ist eine erste Kupplung 124 vorgesehen, die in der Fig. 63 gezeigten Nullstellung geschlossen ist. Wird die Stellhülse 107 gedreht, so wird über den Mitnehmer 113 und die erste Kupplung 124 das Einstellteil 122 mitgenommen. Zwischen dem Bedienelement 106 und dem Rastteil 126 ist eine zweite Kupplung 127 vorgesehen, die ein Kupplungsteil 132 umfasst, und die in der in Fig. 63 gezeigten Nullstellung geöffnet ist. In dieser Stellung kann das Bedienelement 106 gegenüber dem Rastteil 126 gedreht werden. Das Kupplungsteil 132 ist zwischen Halterändern 136 und 137 des Einstellteils 122 axial fest am Einstellteil 122 gehalten. Im Rahmen der Fertigungstoleranzen kann das Kupplungsteil 132 dabei gegenüber dem Einstellteil 122 axial beweglich sein. Das Kupplungsteil 132 ist drehfest mit dem Rastteil 126 verbunden. Über das Rastteil 126 ist das Kupplungsteil 132 drehfest mit dem Gehäuse 102 verbunden. Bei der in Fig. 63 gezeigten geöffneten Stellung der zweiten Kupplung 127 befindet sich das Kupplungsteil 132 vollständig innerhalb des Rastteils 126. Das Kupplungsteil 132 ist in dieser Stellung der zweiten Kupplung 127 gegenüber dem Bedienelement 106 drehbar. Es kann jedoch auch vorteilhaft sein, dass sich das Kupplungsteil 132 in der geöffneten Stellung der Kupplung 127 vollständig innerhalb des Bedienelements 106 befindet und drehfest mit dem Bedienelement 106 verbunden und gegenüber dem Rastteil 126 und dem Gehäuse 102 drehbar ist.

Beim Einstellen einer auszupressenden Menge an Injektionsflüssigkeit dreht der Bediener die Stellhülse 107. Über die drehfeste Verbindung mit dem Einstellteil 122 bewegt sich auch das Einstellteil 122, das aufgrund der dritten Gewindeverbindung 123 auch in Richtung des Pfeils 33 in distale Richtung bewegt wird. Die Feder 128 drückt Rastteil 126 und Bedienelement 106 gegen den Halterand 160 des Einstellteils 122 und führt diese Komponenten bei der distalen Bewegung des Einstellteils 122 nach. Über die drehfeste Verbindung von Mitnehmer 113 und Zustellteil 118 wird das Zustellteil 118 gedreht und bewegt sich aufgrund der ersten Gewindeverbindung 119 in distale Richtung. Der Schieber 120 wird vom Einstellteil 122 mitgenommen und bewegt sich aufgrund der zweiten Gewindeverbindung 121 ebenfalls in distale Richtung.

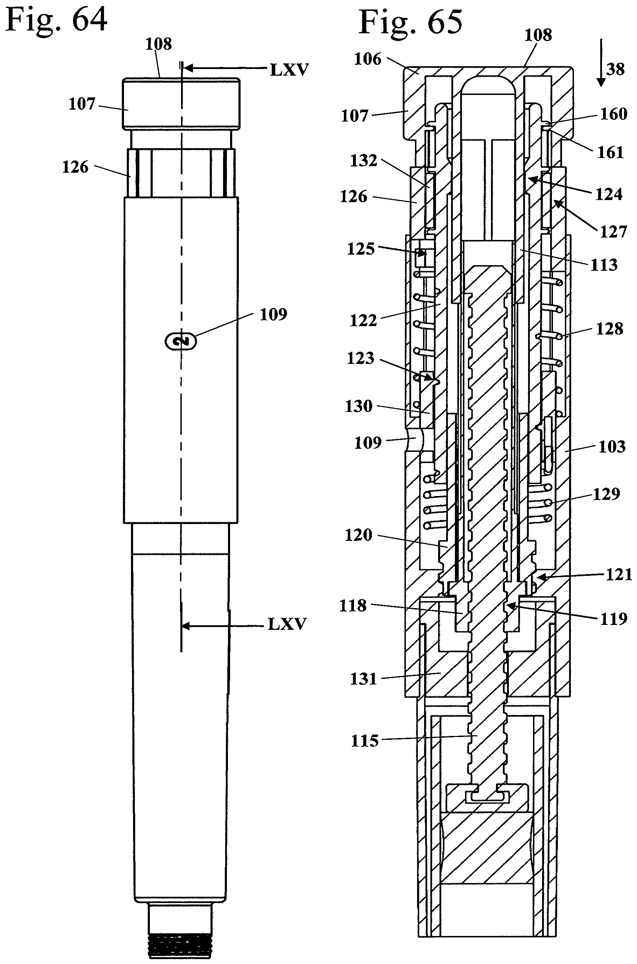

Die Figuren 64 und 65 zeigen die Anordnung nach dem Einstellen der Maximaldosis. Im Sichtfenster 109 ist im Ausführungsbeispiel die Zahl "2" angezeigt. Das Rastteil 126 hat sich teilweise aus dem oberen Gehäuseteil 103 bewegt. Die relative axiale Lage von Einstellteil 122 und Rastteil 126 hat sich nicht geändert, so dass die Rasteinrichtung 125 während des gesamten Einstellvorgangs aktiv ist. Die Feder 129 wird beim Einstellvorgang aufgrund der Relativdrehung von Schieber 120 und Gehäuse 103 gespannt.

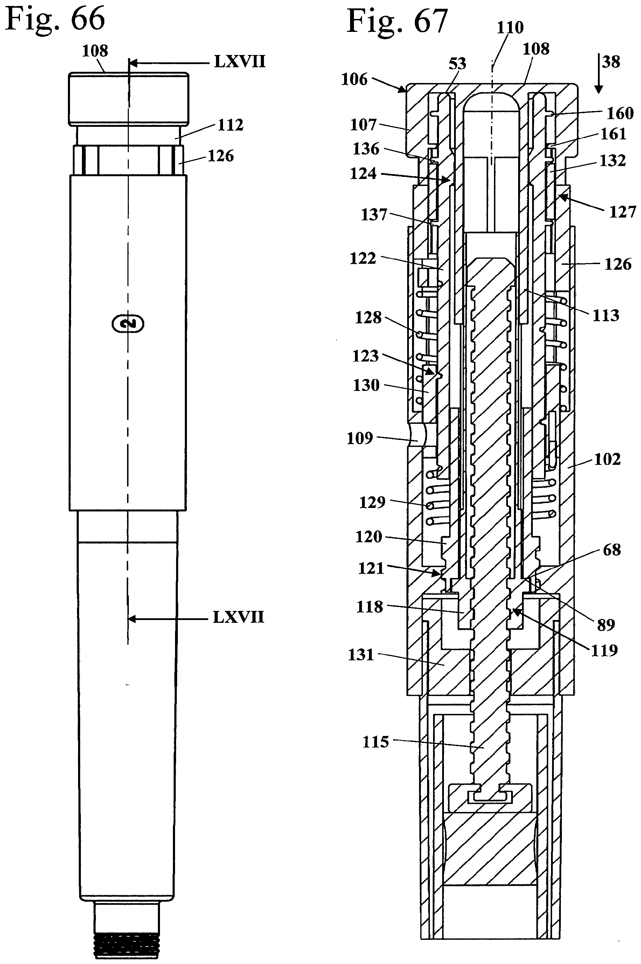

Zum Auspressen der eingestellten Menge an Injektionsflüssigkeit ist der Betätigungsknopf 108 in Richtung des Pfeils 38 in proximale Richtung zu verschieben. Die proximale Position des Betätigungsknopfs 108 ist in den Figuren 66 und 67 gezeigt. Der Betätigungsknopf 108 kann gedrückt werden, bis der Anschlag 53 des Einstellteils 122 am Betätigungsknopf 108 anliegt. Die Halteränder 160 und 161 besitzen in dieser Stellung einen Abstand zueinander. Das Bedienelement 106 besitzt an seiner proximalen Seite einen Druckrand 112, der am Rastteil 126 anliegt. Beim Verschieben des Betätigungsknopfes in proximale Richtung wirkt der Druckrand 112 auf das Rastteil 126 und verschiebt das Rastteil 126 in proximale Richtung. Dadurch kommt der Druckrand 112 in den Bereich des Kupplungsteils 132, und die zweite Kupplung 127 wird geschlossen. Das Kupplungsteil 132 ist in dieser Position sowohl mit dem Bedienelement 106 als auch mit dem Rastteil 126 drehfest verbunden. Dadurch ist der Betätigungsknopf 108 gegenüber dem Gehäuse 102 drehfest gehalten.

Beim Verschieben des Betätigungsknopfes 108 in proximale Richtung wird auch die erste Kupplung 124 zwischen Mitnehmer 113 und Einstellteil 122 gelöst. Dadurch ist das Einstellteil 122 gegenüber Betätigungsknopf 108, Kupplungsteil 132 und Rastteil 126 drehbar. Bei weiterem Verschieben des Betätigungsknopfes 108 wird das Einstellteil 122 in proximale Richtung verschoben. Dabei dreht sich das Einstellteil 122 gegenüber dem Gehäuse 102 aufgrund der dritten Gewindeverbindung 123 und nimmt den Schieber 120 mit, der sich ebenfalls um die Längsmittelachse 110 dreht und in proximale Richtung bewegt, und zwar aufgrund der zweiten Gewindeverbindung 121. Der Schieber 120 wirkt über die Absätze 68 und 89 auf das Zustellteil 118 und verschiebt das Zustellteil 118 in proximale Richtung. Das Zustellteil 118 ist dabei aufgrund der geschlossenen zweiten Kupplung 127 über den Mitnehmer 113, das Bedienelement 106, das Kupplungsteil 132 und das Rastteil 126 drehfest mit dem Gehäuse 102 verbunden. Der Schieber 120 bewegt das Zustellteil 118 zusammen mit der ebenfalls gegenüber dem Gehäuse 102 verdrehgesicherten Kolbenstange 115 in proximale Richtung und drückt dadurch Injektionsflüssigkeit aus dem Behälter 5 aus.

Die zweite Kupplung 127 des Injektionsgeräts 101 verhindert während einer Injektion ein Drehen des Zustellteils 118 gegenüber dem Gehäuse 102. Insoweit ersetzt die zweite Kupplung 127 des Injektionsgeräts 101 die Rasteinrichtung 32 des Injektionsgeräts 1 (siehe beispielsweise Fig. 6).

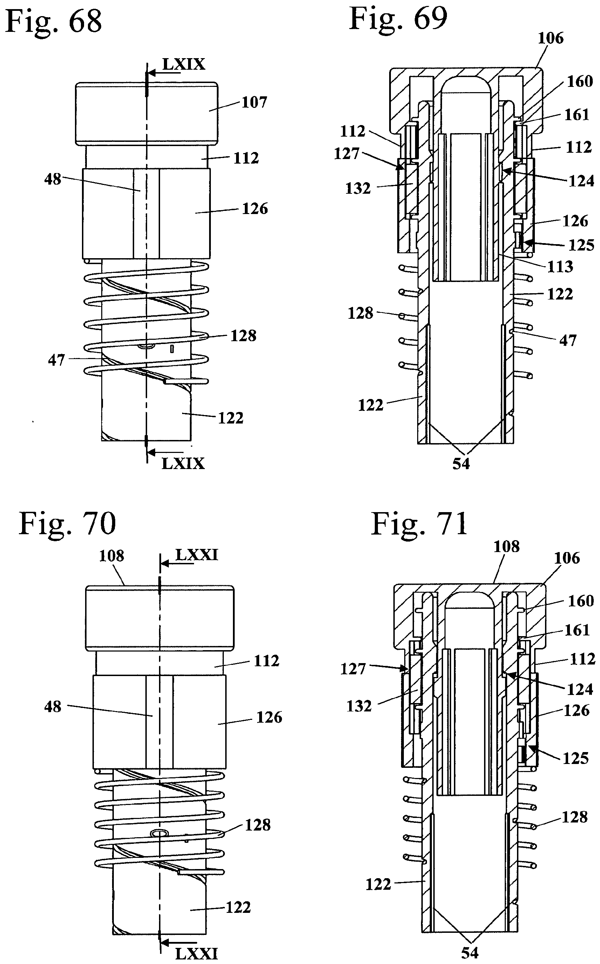

Die Figuren 68 bis 71 zeigen die zweite Kupplung 127 im Einzelnen. Wie Fig. 69 zeigt, ist bei geöffneter Kupplung 127 die Rasteinrichtung 125 aktiv. Die erste Kupplung 124 ist geschlossen. Wie Fig. 71 zeigt, ist in der proximalen Position des Bedienknopfs 108 die erste Kupplung 124 geöffnet und die zweite Kupplung 127 geschlossen. Die Rasteinrichtung 125 ist nicht aktiv, da die Rastelemente an Rastteil 126 und Einstellteil 122 außer Eingriff miteinander sind. Betätigungsknopf 108 und Rastteil 126 sind drehfest miteinander verbunden.

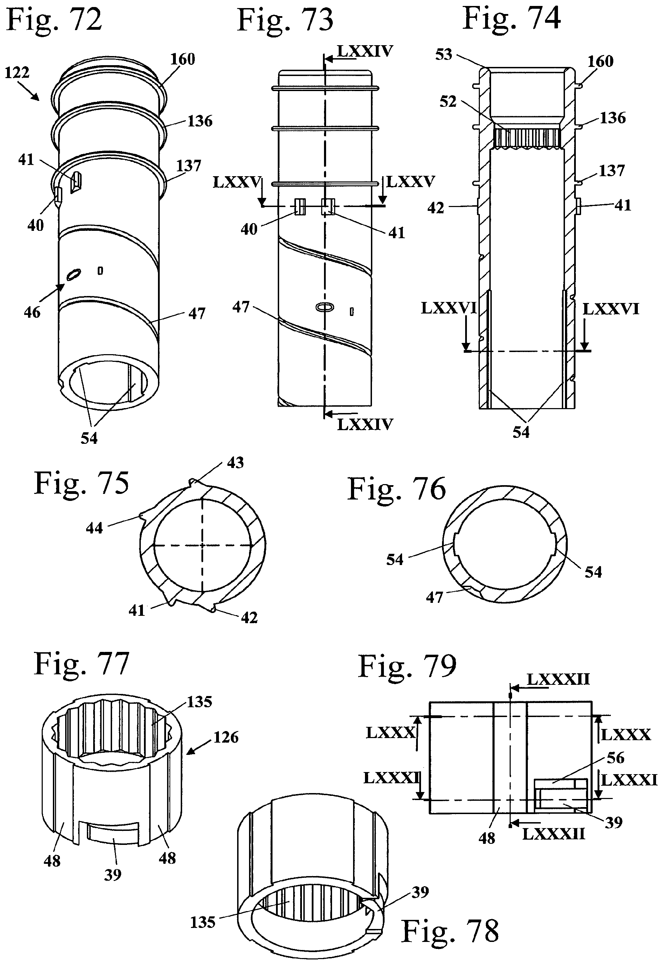

Die Figuren 72 bis 76 zeigen das Einstellteil 122 im Einzelnen. Das Einstellteil 122 besitzt die Rastelemente 41 bis 44. Am Innenumfang des Einstellteils 122 ist die Verzahnung 52 der ersten Kupplung 124 angeordnet.

Die Figuren 77 bis 82 zeigen das Rastteil 126. Das Rastteil 126 unterscheidet sich vom Rastteil 26 durch die Innenverzahnung 135 an der distalen Seite des Rastteils 126.

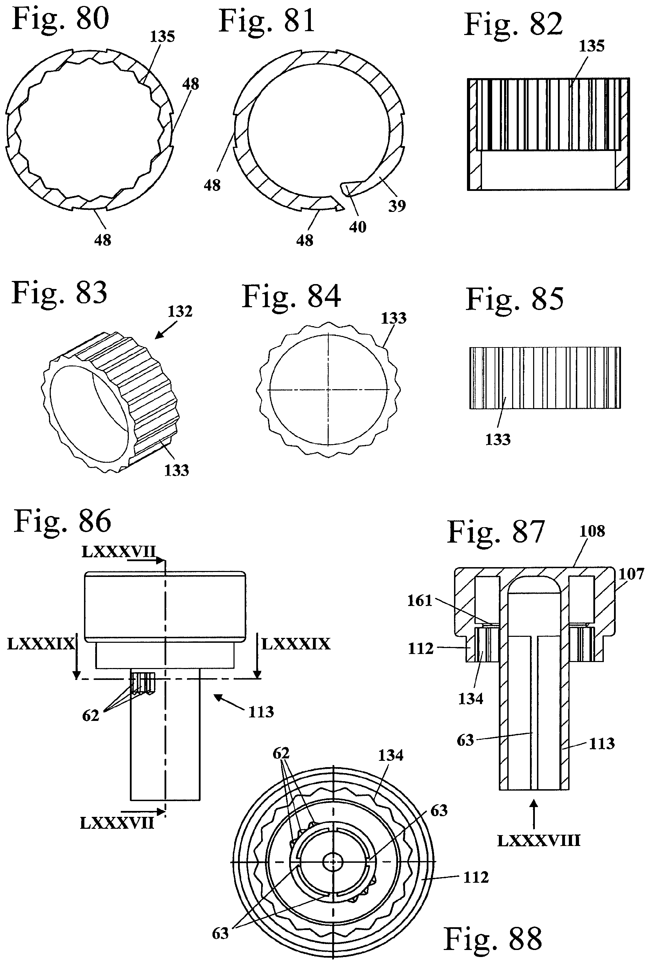

Die Figuren 83 bis 85 zeigen das Kupplungsteil 132. Das Kupplungsteil 132 ist als Ring ausgebildet, der an seinem Außenumfang eine Außenverzahnung 133 besitzt. Die Außenverzahnung 133 wirkt mit der Innenverzahnung 135 des Rastteils 126 zusammen, so dass das Kupplungsteil 132 drehfest im Rastteil 126 gehalten ist.

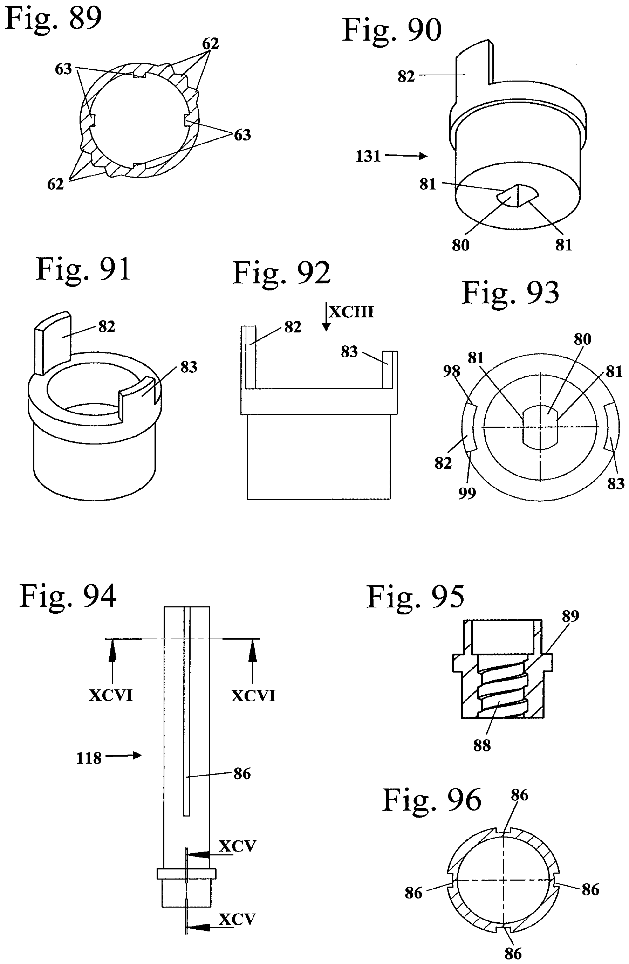

Die Figuren 86 bis 89 zeigen den Mitnehmer 113. Der Mitnehmer 113 besitzt am Druckrand 112 eine Innenverzahnung 134, die der Innenverzahnung 135 des Rastteils 126 entspricht. In der proximalen Position des Betätigungsknopfes 108 ragt das Kupplungsteil 132 in die Innenverzahnung 134 und verbindet dadurch das Rastteil 126 drehfest mit dem Mitnehmer 113. Dadurch ist der Mitnehmer 113 und damit auch das Zustellteil 118 gegenüber dem Gehäuse 102 verdrehgesichert. Wie Fig. 87 zeigt, ist der Halterand 161 an der distalen Seite der Verzahnung 134 angeordnet. Die Figuren 88 und 89 zeigen auch die Längsstege 63 und Zähne 62 des Mitnehmers 113.

Die Figuren 90 bis 93 zeigen die Kolbenführung 131 mit der Öffnung 80. Wie die Figuren 91 und 93 zeigen, ist an der Kolbenführung 131 keine Raststruktur vorgesehen. Zwischen dem Zustellteil 118 und der Kolbenführung 131 ist keine Rasteinrichtung notwendig, da das Zustellteil 118 bei gedrücktem Betätigungsknopf 108 und geschlossener zweiter Kupplung 127 über den Mitnehmer 113 und die zweite Kupplung 127 drehfest mit dem Rastteil 126 und damit mit dem Gehäuse 102 verbunden ist. Ein Verdrehen des Betätigungsknopfes 108 und damit des Schiebers 118 beim Auspressen einer eingestellten Menge an Injektionsflüssigkeit ist dadurch sicher verhindert. Wie die Figuren 94 und 96 zeigen, besitzt das Zustellteil 118 entsprechend keine Rastarme. Das Zustellteil 118 ist hülsenförmig ausgebildet und besitzt die Längsnuten 86 sowie das Innengewinde 88.

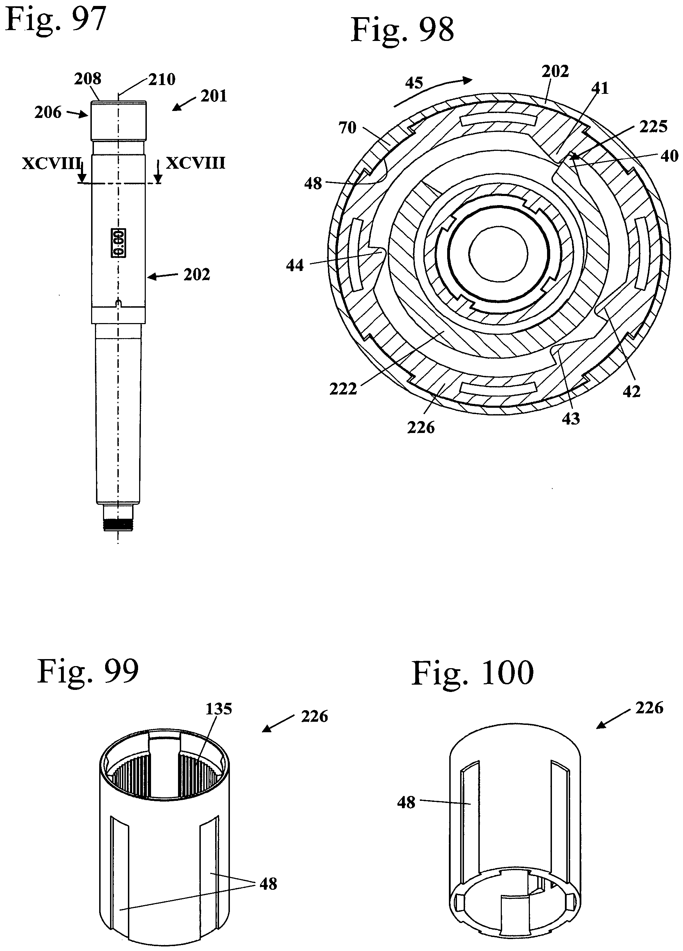

Die Figuren 97 bis 106 zeigen ein Ausführungsbeispiel eines Injektionsgeräts 201, dessen Aufbau im Wesentlichen dem des Injektionsgeräts 101 entspricht. Gleiche Bezugszeichen wie in den voran gegangenen Figuren kennzeichnen gleiche Elemente. Das Injektionsgerät 201 besitzt ein Gehäuse 202 und ein Bedienelement 206. Das Bedienelement 206 besitzt einen Betätigungsknopf 208. Wie Fig. 98 zeigt, ist im Gehäuse 202 ein Rastteil 226 über Längsnuten 48 und Längsstege 70 drehfest und in Richtung seiner Längsmittelachse 210 verschiebbar gehalten. Das Injektionsgerät 201 besitzt außerdem ein Einstellteil 222, das im Ausführungsbeispiel radial innerhalb des Rastteils 226 angeordnet ist. Zwischen dem Rastteil 226 und dem Einstellteil 222 wirkt eine Rasteinrichtung 225. Die Rasteinrichtung 225 umfasst ein am Einstellteil 222 ausgebildetes Rastelement 40, das an einem Rastarm 39 federnd gehalten ist, sowie Rastelemente 41, 42, 43 und 44 am Rastteil 226.

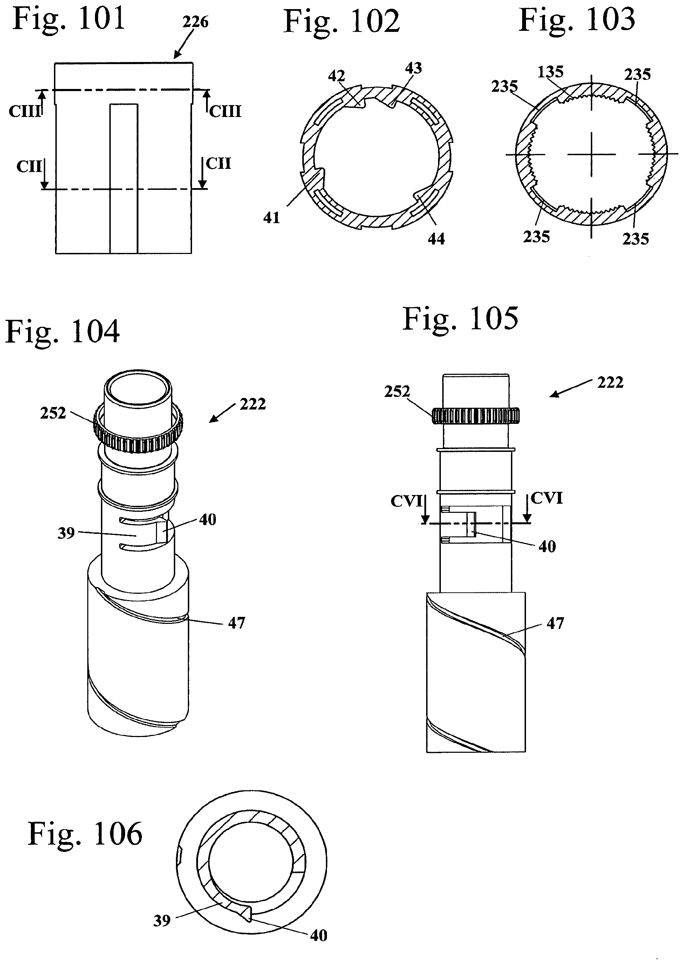

Die Figuren 99 bis 103 zeigen die Gestaltung des Rastteils 226 im Einzelnen. In seinem distalen Bereich besitzt das Rastteil 226 eine Innenverzahnung 135 zur drehfesten Verbindung mit einer Außenverzahnung 133 eines Kupplungsteils 132. Im proximalen Bereich sind am Innenumfang des Rastteils 226 die Rastelemente 41 bis 44 angeordnet, wie die Figuren 100 und 102 zeigen. Wie Fig. 102 zeigt, sind die Rastelemente 41 bis 44 als rampenförmige Erhebungen am Innenumfang des Rastteils 226 ausgebildet. Fig. 103 zeigt die Innenverzahnung 135, die im Ausführungsbeispiel durch mehrere Unterbrechungen 235 unterbrochen ist.

Die Figuren 104 bis 106 zeigen das Einstellteil 222 im Einzelnen. Das Einstellteil 222 trägt im proximalen Bereich an seinem Außenumfang ein Außengewinde 47. In seinem distalen Bereich besitzt das Einstellteil 222 eine Verzahnung 252. Die Verzahnung 252 ist Teil einer Kupplung, die der Kupplung 124 entspricht, und dient zur drehfesten Verbindung mit dem Bedienelement 206. Das Bedienelement 206 trägt eine nicht gezeigte, der Verzahnung 252 zugeordnete Verzahnung an seiner Innenseite. In einem mittleren Bereich, dessen Außendurchmesser gegenüber dem Außendurchmesser des Außengewindes 47 deutlich verringert ist, ist der Rastarm 39 mit dem Rastelement 40 vorgesehen.

Die Funktion des Injektionsgeräts 201 entspricht der zum Injektionsgerät 101 beschriebenen Funktion. Durch die Anordnung der festen Rastelemente 41 bis 44 am Rastteil 226 und die Anordnung des Rastarms 39 mit dem Rastelement 40 am Einstellteil 222 ist die Entformung des Einstellteils 222 aus einem Spritzgusswerkzeug bei der Herstellung des Einstellteils 222 vereinfacht. Werden andere Dosiseinstellungen und damit andere Raststellungen für ein Injektionsgerät 201 gewünscht, so muss lediglich die Position der Rastelemente 41 bis 44 am Rastteil 226 geändert werden. Das aufwändiger herzustellende Einstellteil 222 kann unverändert bleiben.

Beim Injektionsgerät 201 ist der Anschlag für die in den Figuren 97 und 98 gezeigte Nullstellung zwischen dem Rastelement 40 und dem Rastelement 41 gebildet. Es kann jedoch auch vorgesehen sein, den Anschlag für die Nullstellung nicht zwischen Rastteil 226 und Einstellteil 222 auszubilden, sondern zwischen dem Schieber 120 (Fig. 63) und dem Gehäuse 202. Der Anschlag kann beispielsweise durch eine axiale Erhebung am Schieber 120 gebildet sein, die mit einer entsprechenden Erhebung oder einer Aussparung am Gehäuse 201 zusammenwirkt.

Bei den Injektionsgeräten 1, 101 und 201 wirkt die Rasteinrichtung 25, 125, 225 jeweils zwischen dem drehfest im Gehäuse 2, 102, 202 gehaltenen Rastteil 26, 26', 126, 226 und dem Einstellteil 22, 22', 122, 222, das sich beim Einstellen der Dosis in der ersten Drehrichtung 45 und beim Auspressen der Dosis in entgegen gerichteter Drehrichtung 51 dreht. Dadurch, dass sich das Einstellteil 22, 22', 122, 222 beim Auspressen der Dosis zurückdreht, ist jeder Raststellung eine definierte Stellung des Einstellteils 22, 22', 122, 222 gegenüber dem Rastteil 26, 126, 226 zugeordnet. Dadurch können unterschiedliche Abstände zwischen den Raststellungen vorgesehen werden. Zum Einstellen einer Dosis muss der Bediener lediglich vorgesehene Raststellungen überdrücken. Lediglich bei der beim Injektionsgerät 1, nicht aber bei den Injektionsgeräten 101, 201 vorgesehenen zweiten Rasteinrichtung 32 sind zusätzlich Zwischenschritte einer Rasteinrichtung zu überwinden, deren Widerstand jedoch deutlich geringer als der der Rasteinrichtung 25 ist. Dadurch ergibt sich eine ergonomische, einfache Bedienung.

## Patentansprüche

1. Injektionsgerät mit einem Gehäuse (2, 102, 202), mit einem Dosierkolben (14, 114) zum Auspressen von Injektionsflüssigkeit aus einem Behälter (5, 105), mit einem Zustellteil (18, 118), das über eine erste Gewindeverbindung (19, 119) mit dem Dosierkolben (14, 114) verbunden ist, mit einem Schieber (20, 120), der ein Gewinde einer zweiten Gewindeverbindung (21, 121) trägt, und mit einem Einstellteil (22, 22', 122, 222), das ein Gewinde einer dritten Gewindeverbindung (23, 123) trägt, wobei das Einstellteil (22, 22', 122, 222) sich beim Einstellen einer aus dem Injektionsgerät (1, 101, 201) auszupressenden Menge an Injektionsflüssigkeit gegenüber dem Gehäuse (2, 102, 202) in einer ersten Drehrichtung (45) um eine Längsmittelachse (10, 110, 210) des Injektionsgeräts (1, 101, 201) dreht und aufgrund der dritten Gewindeverbindung (23, 123) in distaler Richtung bewegt und wobei das Einstellteil (22, 22', 122, 222) sich beim Auspressen der Injektionsflüssigkeit aus dem Behälter (5, 105) in einer der ersten Drehrichtung (45) entgegen gerichteten zweiten Drehrichtung (51) dreht und in proximaler Richtung bewegt, und mit einer Rasteinrichtung (25, 125, 225), die mindestens eine Raststellung des Einstellteils (22, 22', 122, 222) definiert, wobei die Rasteinrichtung (25, 125, 225) zwischen dem Einstellteil (22, 22', 122, 222) und dem Gehäuse (2, 102, 202) wirkt, und wobei die Rasteinrichtung (25, 125, 225) mindestens beim Einstellen der aus dem Behälter (5, 105) auszupressenden Menge an Injektionsflüssigkeit wirksam ist,
**dadurch gekennzeichnet, dass** jeder Raststellung eine eindeutige Drehlage des Einstellteils (22, 22', 122, 222) gegenüber dem Gehäuse (2, 102, 202) zugeordnet ist.

2. Injektionsgerät nach Anspruch 1,
**dadurch gekennzeichnet, dass** die Rasteinrichtung (25, 125, 225) ein Rastteil (26, 26', 126, 226) umfasst, das unabhängig vom Einstellteil (22, 22', 122, 222) in Richtung der Längsmittelachse (10, 110, 210) verschiebbar ist und das drehfest mit dem Gehäuse (2, 102, 202) verbunden ist, wobei an dem Rastteil (26, 26', 126, 226) mindestens ein erstes Rastelement (40, 41, 42, 43, 44, 93, 94, 95, 96) der Rasteinrichtung (25, 125, 225) angeordnet ist.

3. Injektionsgerät nach Anspruch 2,
**dadurch gekennzeichnet, dass** an dem Einstellteil (22, 22', 122, 222) mindestens ein zweites Rastelement (40, 41, 42, 43, 44, 92) angeordnet ist, wobei das mindestens eine erste Rastelement (40, 41, 42, 43, 44,93, 94, 95, 96) und das mindestens eine zweite Rastelement (40, 41, 42, 43, 44, 92) in einer ersten axialen Stellung von Rastteil (26, 26', 126, 226) und Einstellteil (22, 22', 122, 222) die mindestens eine Raststellung definieren und in mindestens einer zweiten axialen Stellung von Rastteil (26, 26', 126, 226) und Einstellteil (22, 22', 122, 222) unabhängig von der relativen Drehlage des Einstellteils (22, 22', 122, 222) zum Rastteil (26, 26', 126, 226) außer Eingriff sind.

4. Injektionsgerät nach Anspruch 3,
**dadurch gekennzeichnet, dass** das Injektionsgerät (1, 101, 201) eine Feder (28, 128) besitzt, die das Rastteil (26, 26', 126, 226) in Richtung auf die erste axiale Stellung vorspannt.

5. Injektionsgerät nach einem der Ansprüche 2 bis 4,
**dadurch gekennzeichnet, dass** das Einstellteil (22, 22', 122, 222) drehfest mit dem Schieber (20, 120) verbunden ist, dass der Schieber (20, 120) beim Auspressen von Injektionsflüssigkeit aus dem Behälter (5, 105) derart auf das Zustellteil (18, 118) wirkt, dass der Schieber (20, 120) bei einer Bewegung in proximaler Richtung das Zustellteil (18, 118) in proximaler Richtung verschiebt und dass der Dosierkolben (14, 114) drehfest im Gehäuse (2, 102, 202) gehalten ist.

6. Injektionsgerät nach einem der Ansprüche 2 bis 5,
**dadurch gekennzeichnet, dass** das Injektionsgerät (1, 101, 201) einen Mitnehmer (13, 113) besitzt, der drehfest mit dem Zustellteil (18, 118) verbunden ist, dass das Injektionsgerät (1, 101, 201) eine Kupplung (24, 124) besitzt, die in einer ersten Stellung das Einstellteil (22, 22', 122, 222) drehfest mit dem Mitnehmer (13, 113) verbindet und die in einer zweiten Stellung eine Relativdrehung des Einstellteils (22, 22', 122, 222) gegenüber dem Mitnehmer (13, 113) zulässt und dass das Verstellen der Kupplung (24, 124) von der ersten Stellung in die zweite Stellung durch Verschieben eines Betätigungsknopfes (8, 108, 208) des Injektionsgerätes (1, 101, 201) in proximaler Richtung erfolgt.

7. Injektionsgerät nach Anspruch 6,
**dadurch gekennzeichnet, dass** das Rastteil (26, 26', 126, 226) in Richtung der Längsmittelachse (10,110, 210) derart an dem Betätigungsknopf (8, 108, 208) gekoppelt ist, dass eine Bewegung des Betätigungsknopfes (8, 108, 208) in proximaler Richtung eine Bewegung des Rastteils (26, 26', 126, 226) in proximaler Richtung bewirkt.

8. Injektionsgerät nach Anspruch 7,
**dadurch gekennzeichnet, dass** das Injektionsgerät (1) eine Stellhülse (7) zum Einstellen der auszupressenden Menge an Injektionsflüssigkeit besitzt, die fest mit dem Einstellteil (22, 22') verbunden ist.

9. Injektionsgerät nach Anspruch 8,
**dadurch gekennzeichnet, dass** der Betätigungsknopf (8) über ein Druckstück (27) auf das Rastteil (26, 26') wirkt, wobei das Druckstück (27) drehbar gegenüber dem Betätigungsknopf (8) gelagert ist und drehfest mit dem Einstellteil (22, 22') verbunden ist.

10. Injektionsgerät nach Anspruch 9,
**dadurch gekennzeichnet, dass** das Einstellteil (22, 22') mit der Stellhülse (7) über einen Ringsteg (34) verbunden ist, der mindestens eine Öffnung (55) besitzt, durch die ein Drucksteg (37) des Druckstücks (27) ragt.

11. Injektionsgerät nach einem der Ansprüche 1 bis 10,
**dadurch gekennzeichnet, dass** das Zustellteil (18) über eine zweite Rasteinrichtung (32) mit dem Gehäuse (2) verbunden ist, wobei die zweite Rasteinrichtung (32) mindestens einen Längssteg (85) umfasst, an dem das Zustellteil (18) beim Auspressen von Injektionsflüssigkeit geführt ist.

12. Injektionsgerät nach Anspruch 6 oder 7,
**dadurch gekennzeichnet, dass** das Injektionsgerät (101, 201) eine Stellhülse (107) zum Einstellen der auszupressenden Menge an Injektionsflüssigkeit besitzt, die fest mit dem Betätigungsknopf (108, 208) verbunden ist.

13. Injektionsgerät nach Anspruch 12,
**dadurch gekennzeichnet, dass** das Injektionsgerät (101, 201) eine zweite Kupplung (127) besitzt, die in der distalen Position des Betätigungsknopfes (108, 208) eine Relativdrehung des Mitnehmers (113) gegenüber dem Rastteil (126, 226) erlaubt und die in einer proximalen Position des Betätigungsknopfes (108, 208) den Mitnehmer (113) drehfest mit dem Rastteil (126, 226) verbindet.

14. Injektionsgerät nach Anspruch 13,
**dadurch gekennzeichnet, dass** der Mitnehmer (113) und das Rastteil (126, 226) jeweils eine Verzahnung besitzen und dass am Einstellteil (122, 222) ein Kupplungsteil (132) angeordnet ist, das eine Gegenverzahnung trägt, wobei die Gegenverzahnung in der distalen Position des Betätigungsknopfes (108, 208) nur mit einer der Verzahnungen von Mitnehmer (113) und Rastteil (126, 226) zusammenwirkt und in der proximalen Position des Betätigungsknopfes (108, 208) sowohl mit der Verzahnung am Mitnehmer (113) als auch mit der Verzahnung am Rastteil (126, 226) drehfest verbunden ist.

15. Injektionsgerät nach Anspruch 14,
**dadurch gekennzeichnet, dass** das Kupplungsteil (132) gegenüber dem Einstellteil (122, 222) drehbar ist, und dass das Kupplungsteil (132) in Richtung der Längsmitteilachse (110, 210) ortsfest am Einstellteil (122, 222) gehalten ist.

16. Injektionsgerät nach einem der Ansprüche 1 bis 15,
**dadurch gekennzeichnet, dass** das Injektionsgerät (1, 101, 201) eine Feder (29, 129) besitzt, die zwischen dem Schieber (20, 120) und dem Gehäuse (2, 102, 202) wirkt und die den Schieber (20, 120) in der zweiten Drehrichtung (51) vorspannt.

## Claims

1. Injection device with a housing (2, 102, 202), with a dosing piston (14, 114) for expressing injection fluid from a container (5, 105), with a feed part (18, 118) connected to the dosing piston (14, 114) via a first threaded connection (19, 119), with a slide (20, 120) supporting a thread of a second threaded connection (21, 121), and with a setting part (22, 22', 122, 222) supporting a thread of a third threaded connection (23, 123), wherein the setting part (22, 22', 122, 222), when setting a quantity of injection fluid to be expressed from the injection device (1, 101, 201), rotates with respect to the housing (2, 102, 202) in a first direction of rotation (45) about a longitudinal central axis (10, 110, 210) of the injection device (1, 101, 201) and moves in the distal direction by virtue of the third threaded connection (23, 123), and wherein the setting part (22, 22', 122, 222),, when expressing the injection fluid from the container (5, 105), rotates in a second direction of rotation (51) counter to the first direction of rotation (45) and moves in the proximal direction, and with a latching device (25, 125, 225) defining at least one latching position of the setting part (22, 22', 122, 222), wherein the latching device (25, 125, 225) acts between the setting part (22, 22', 122, 222) and the housing (2, 102, 202) and wherein the latching device (25, 125, 225) is active at least when setting the quantity of injection fluid to be expressed from the container (5, 105),
**characterised in that** each latching position is assigned an unequivocal rotational position of the setting part (22, 22', 122, 222) with respect to the housing (2, 102, 202).

2. Injection device according to claim 1,
**characterised in that** the latching device (25, 125, 225) comprises a latching part (26, 26', 126, 226), which is displaceable in the direction of the longitudinal central axis (10, 110, 210) independently of the setting part (22, 22', 122, 222) and which is non-rotatably connected to the housing (2, 102, 202), wherein at least one first latching element (40, 41, 42, 43, 44, 93, 94, 95, 96) of the latching device (25, 125, 225) is located on the latching part (26, 26', 126, 226).

3. Injection device according to claim 2,
**characterised in that** at least one second latching element (40, 41, 42, 43, 44, 93) is located on the setting part (22, 22', 122, 222), wherein the at least one first latching element (40, 41, 42, 43, 44, 93, 94, 95, 96) and the at least one second latching element (40, 41, 42, 43, 44, 93) define in a first axial position of the latching part (26, 26', 126, 226) and the setting part (22, 22', 122, 222) the at least one latching position and are in at least one second axial position of the latching part (26, 26', 126, 226) and the setting part (22, 22', 122, 222) out of engagement independently of the rotational position of the setting part (22, 22', 122, 222) relative to the latching part (26, 26', 126, 226).

4. Injection device according to claim 3,
**characterised in that** the injection device (1, 101, 201) has a spring (28, 128), which preloads the latching part (26, 26', 126, 226) towards the first axial position.

5. Injection device according to any of claims 2 to 4,
**characterised in that** the setting part (22, 22', 122, 222) is non-rotatably connected to the slide (20, 120), **in that** the slide (20, 120), when expressing injection fluid from the container (5, 105), acts on the feed part (18, 118) in such a way that the slide (20, 120), when moving in the proximal direction, displaces the feed part (18, 118) in the proximal direction, and **in that** the dosing piston (14, 114) is non-rotatably held in the housing (2, 102, 202).

6. Injection device according to any of claims 2 to 5,
**characterised in that** the injection device (1, 101, 201) has an entrainer (13, 113), which is non-rotatably connected to the feed part (18, 118), **in that** the injection device (1, 101, 201) has a coupling (24, 124), which is non-rotatably connects the setting part (22, 22', 122, 222) to the entrainer (13, 113) in a first position and permits a rotation of the setting part (22, 22', 122, 222) relative to the entrainer (13, 113) in a second position, and **in that** the coupling (24, 124) is moved from the first position to the second position by displacing an actuation button (8, 108, 208) of the injection device (1, 101, 201) in the proximal direction.

7. Injection device according to claim 6,
**characterised in that** the latching part (26, 26', 126, 226) is coupled to the actuation button (8, 108, 208) in the direction of the longitudinal central axis (10, 110, 210) in such a way that a movement of the actuation button (8, 108, 208) in the proximal direction causes a movement of the latching part (26, 26', 126, 226) in the proximal direction.

8. Injection device according to claim 7,
**characterised in that** the injection device (1) has an adjustment sleeve (7) permanently connected to the setting part (22, 22') for setting the quantity of injection fluid to be expressed.

9. Injection device according to claim 8,
**characterised in that** the actuation button (8) acts on the latching part (26, 26') by way of a pressure member (27), wherein the pressure member (27) is rotatable with respect to the actuation button (8) and non-rotatably connected to the setting part (22, 22').

10. Injection device according to claim 9,
**characterised in that** the setting part (22, 22') is connected to the adjustment sleeve (7) via an annular web (34), which has at least one opening (55), through which a pressure web (37) of the pressure member (27) protrudes.

11. Injection device according to any of claims 1 to 10,
**characterised in that** the feed part (18) is connected to the housing (2) via a second latching device (32), wherein the second latching device (32) comprises at least one longitudinal web (85), along which the feed part (18) is guided when expressing injection fluid.

12. Injection device according to claim 6 or 7,
**characterised in that** the injection device (101, 201) has an adjustment sleeve (107) permanently connected to the actuation button (108, 208) for setting the quantity of injection fluid to be expressed.

13. Injection device according to claim 12,
**characterised in that** the injection device (101, 201) has a second coupling (127), which permits a rotation of the entrainer (113) relative to the latching part (126, 226) in the distal position of the actuation button (108, 208) and connects the entrainer (113) non-rotatably to the latching part (126, 226) in a proximal position of the actuation button (108, 208).

14. Injection device according to claim 13,
**characterised in that** the entrainer (113) and the latching part (126, 226) have a toothing each, and **in that** a coupling part (132) having a mating toothing is located on the setting part (122, 222), wherein the mating toothing acts together with only one of the toothings of the entrainer (113) and the latching part (126, 226) in the distal position of the actuation button (108, 208) and is non-rotatably connected both to the toothing on the entrainer (113) and to the toothing on the latching part (126, 226) in the proximal position of the actuation button (108, 208).

15. Injection device according to claim 14,
**characterised in that** the coupling part (132) is rotatable with respect to the setting part (122, 222), and **in that** the coupling part (132) is held stationary on the setting part (122, 222) in the direction of the longitudinal central axis (110, 210).

16. Injection device according to any of claims 1 to 15,
**characterised in that** the injection device (1, 101, 201) has a spring (29, 129), which acts between the slide (20, 120) and the housing (2, 102, 202) and preloads the slide (20, 120) in the second direction of rotation (51).

## Revendications

1. Appareil d'injection avec un boîtier (2, 102, 202), avec un piston de dosage (14, 114) servant à expulser du liquide d'injection hors d'un contenant (5, 105), avec une partie de distribution (18, 118) qui est reliée au piston de dosage (14, 114) par l'intermédiaire d'un premier raccord fileté (19, 119), avec un coulisseau (20, 120) qui supporte un filetage d'un deuxième raccord fileté (21, 121), et avec une partie de réglage (22, 22', 122, 222) qui supporte un filetage d'un troisième raccord fileté (23, 123), dans lequel la partie de réglage (22, 22', 122, 222) tourne lors du réglage d'une quantité de liquide d'injection à expulser hors de l'appareil d'injection (1, 101, 201) par rapport au boîtier (2, 102, 202) dans une première direction de rotation (45) autour d'un axe central longitudinal (10, 110, 210) de l'appareil d'injection (1, 101, 201) et se déplace dans une direction distale du fait du troisième raccord fileté (23, 123), et dans lequel la partie de réglage (22, 22', 122, 222) tourne lors de l'expulsion du liquide d'injection hors du contenant (5, 105) dans une deuxième direction de rotation (51) dirigée à l'opposé de la première direction de rotation (45) et se déplace dans une direction proximale, et avec un dispositif d'enclenchement (25, 125, 225) qui définit au moins une position d'enclenchement de la partie de réglage (22, 22', 122, 222), dans lequel le dispositif d'enclenchement (25, 125, 225) agit entre la partie de réglage (22, 22', 122, 222) et le boîtier (2, 102, 202), et dans lequel le dispositif d'enclenchement (25, 125, 225) est actif au moins lors du réglage de la quantité de liquide d'injection à expulser hors du contenant (5, 105), **caractérisé en ce qu'**une position de rotation claire de la partie de réglage (22, 22', 122, 222) par rapport au boîtier (2, 102, 202) est associée à chaque position d'enclenchement.

2. Appareil d'injection selon la revendication 1,
**caractérisé en ce que** le dispositif d'enclenchement (25, 125, 225) comprend une partie d'enclenchement (26, 26', 126, 226) qui peut être coulissée indépendamment de la partie de réglage (22, 22', 122, 222) en direction de l'axe central longitudinal (10, 110, 210) et est reliée de manière solidaire en rotation au boîtier (2, 102, 202), dans lequel au moins un premier élément d'enclenchement (40, 41, 42, 43, 44, 93, 94, 95, 96) du dispositif d'enclenchement (25, 125, 225) est disposé sur la partie d'enclenchement (26, 26', 126, 226).

3. Appareil d'injection selon la revendication 2,
**caractérisé en ce qu'**au moins un deuxième élément d'enclenchement (40, 41, 42, 43, 44, 92) est disposé sur la partie de réglage (22, 22', 122, 222), dans lequel l'au moins un premier élément d'enclenchement (40, 41, 42, 43, 44, 93, 94, 95, 96) et l'au moins un deuxième élément d'enclenchement (40, 41, 42, 43, 44, 92) définissent dans une première position axiale de la partie d'enclenchement (26, 26', 126, 226) et de la partie de réglage (22, 22', 122, 222) l'au moins une position d'enclenchement et ne sont pas en prise dans au moins une deuxième position axiale de la partie d'enclenchement (26, 26', 126, 226) et de la partie de réglage (22, 22', 122, 222) indépendamment de la position de rotation relative de la partie de réglage (22, 22', 122, 222) par rapport à la partie d'enclenchement (26, 26', 126, 226).

4. Appareil d'injection selon la revendication 3,
**caractérisé en ce que** l'appareil d'injection (1, 101, 201) possède un ressort (28, 128) qui précontraint la partie d'enclenchement (26, 26', 126, 226) en direction de la première position axiale.

5. Appareil d'injection selon l'une des revendications 2 à 4,
**caractérisé en ce que** la partie de réglage (22, 22', 122, 222) est reliée de manière solidaire en rotation au coulisseau (20, 120), que le coulisseau (20, 120) agit lors de l'expulsion du liquide d'injection hors du contenant (5, 105) sur la partie de distribution (18, 118) de telle manière que le coulisseau (20, 120) fasse coulisser la partie de distribution (18, 118) dans une direction proximale lors d'un déplacement dans une direction proximale, et que le piston de dosage (14, 114) est maintenu de manière solidaire en rotation dans le boîtier (2, 102, 202).

6. Appareil d'injection selon l'une des revendications 2 à 5,
**caractérisé en ce que** l'appareil d'injection (1, 101, 201) possède un entraîneur (13, 113) qui est relié de manière solidaire en rotation à la partie de distribution (18, 118), que l'appareil d'injection (1, 101, 201) possède un couplage (24, 124) qui relie dans une première position la partie de réglage (22, 22', 122, 222) de manière solidaire en rotation à l'entraîneur (13, 113) et qui autorise dans une deuxième position une rotation relative de la partie de réglage (22, 22', 122, 222) par rapport à l'entraîneur (13, 113), et que l'ajustement du couplage (24, 124) de la première position à la deuxième position est effectué par le coulissement d'un bouton d'actionnement (8, 108, 208) de l'appareil d'injection (1, 101, 201) dans une direction proximale.

7. Appareil d'injection selon la revendication 6,
**caractérisé en ce que** la partie d'enclenchement (26, 26', 126, 226) est couplée en direction de l'axe central longitudinal (10, 110, 210) au bouton d'actionnement (8, 108, 208) de telle manière qu'un déplacement du bouton d'actionnement (8, 108, 208) dans une direction proximale provoque un déplacement de la partie d'enclenchement (26, 26', 126, 226) dans une direction proximale.

8. Appareil d'injection selon la revendication 7,
**caractérisé en ce que** l'appareil d'injection (1) possède une douille de réglage (7) servant à régler la quantité de liquide d'injection à expulser, qui est reliée de manière solidaire à la partie de réglage (22, 22').

9. Appareil d'injection selon la revendication 8,
**caractérisé en ce que** le bouton d'actionnement (8) agit par l'intermédiaire d'une pièce de pression (27) sur la partie d'enclenchement (26, 26'), dans lequel la pièce de pression (27) est montée à rotation par rapport au bouton d'actionnement (8) et est reliée de manière solidaire en rotation à la partie de réglage (22, 22').

10. Appareil d'injection selon la revendication 9,
**caractérisé en ce que** la partie de réglage (22, 22') est reliée à la douille de réglage (7) par l'intermédiaire d'une entretoise annulaire (34) qui possède au moins une ouverture (55) par laquelle une entretoise de pression (37) de la pièce de pression (27) dépasse.

11. Appareil d'injection selon l'une des revendications 1 à 10,
**caractérisé en ce que** la partie de distribution (18) est reliée au boîtier (2) par l'intermédiaire d'un deuxième dispositif d'enclenchement (32), dans lequel le deuxième dispositif d'enclenchement (32) comprend au moins une entretoise longitudinale (85) contre laquelle la partie de distribution (18) est guidée lors de l'expulsion du liquide d'injection.

12. Appareil d'injection selon la revendication 6 ou 7,
**caractérisé en ce que** l'appareil d'injection (101, 201) possède une douille de réglage (107) servant à régler la quantité de liquide d'injection à expulser, qui est reliée de manière solidaire au bouton d'actionnement (108, 208).

13. Appareil d'injection selon la revendication 12,
**caractérisé en ce que** l'appareil d'injection (101, 201) possède un deuxième couplage (127) qui permet dans la position distale du bouton d'actionnement (108, 208) une rotation relative de l'entraîneur (113) par rapport à la partie d'enclenchement (126, 226) et qui relie, dans une position proximale du bouton d'actionnement (108, 208), l'entraîneur (113) de manière solidaire en rotation à la partie d'enclenchement (126, 226).

14. Appareil d'injection selon la revendication 13,
**caractérisé en ce que** l'entraîneur (113) et la partie d'enclenchement (126, 226) possèdent respectivement une denture, et qu'une partie de couplage (132) est disposée sur la partie de réglage (122, 222), laquelle supporte une contre-denture, dans lequel la contre-denture coopère dans la position distale du bouton d'actionnement (108, 208) seulement avec une des dentures de l'entraîneur (113) et de la partie d'enclenchement (126, 226) et est reliée dans la position proximale du bouton d'actionnement (108, 208) de manière solidaire en rotation à la fois à la denture sur l'entraîneur (113) et à la denture sur la partie d'enclenchement (126, 226).

15. Appareil d'injection selon la revendication 14,
**caractérisé en ce que** la partie de couplage (132) peut tourner par rapport à la partie de réglage (122, 222), et que la partie de couplage (132) est maintenue de manière stationnaire sur la partie de réglage (122, 222) en direction de l'axe central longitudinal (110, 210).

16. Appareil d'injection selon l'une des revendications 1 à 15,
**caractérisé en ce que** l'appareil d'injection (1, 101, 201) possède un ressort (29, 129) qui agit entre le coulisseau (20, 120) et le boîtier (2, 102, 202) et qui précontraint le coulisseau (20, 120) dans la deuxième direction de rotation (51).
